# EUROPÄISCHE PATENTSCHRIFT

(11) **EP 1 805 316 B1**
(45) Veröffentlichungstag und Bekanntmachung des Hinweises auf die Patenterteilung: **15.04.2009**
(21) Anmeldenummer: 05778085.0
(22) Anmeldetag: 23.07.2005
(51) Int. Cl.: C12P 17/00, C12P 7/24, C12P 41/00

(54) **VERFAHREN ZUR HERSTELLUNG DER ENANTIOMEREN FORMEN VON CIS-3-HYDROXYCYCLOHEXANCARBONSÄURE-DERIVATEN UNTER VERWENDUNG VON HYDROLASEN**
PROCESS FOR THE PREPARATION OF THE ENANTIOMERIC FORMS OF CIS-3-HYDROXYCYCLOHEXANE CARBOXYLIC ACID DERIVATIVES EMPLOYING HYDROLASES
PROCÉDÉ DE PRÉPARATION DES FORMES ÉNANTIOMÈRES DE DÉRIVÉS DE L'ACIDE CIS-3-HYDROXYCYCLOHEXANE CARBOXYLIQUE EN UTILISANT DES HYDROLASES

(30) Priorität: 07.08.2004 DE 102004038403
(43) Veröffentlichungstag der Anmeldung: 11.07.2007
(73) Patentinhaber: Sanofi-Aventis Deutschland GmbH, 65929 Frankfurt am Main (DE)
(72) Erfinder: HOLLA, Wolfgang, 65779 Kelkheim (DE); KEIL, Stefanie, 65719 Hofheim (DE); TAPPERTZHOFEN, Christoph, 65926 Frankfurt (DE)
(86) Internationale Anmeldenummer: PCT/EP2005/008058
(87) Internationale Veröffentlichungsnummer: WO 2006/015716

(56) Entgegenhaltungen:
- WO-A-03/020269
- WO-A-20/04076390
- WO-A-20/04076426
- LEVY, L.M. ET AL.: "Lipase-catalysed resolution of cyclic cis- and trans-beta-hydroxy esters" TETRAHEDRON: ASYMMETRY, Bd. 14, Nr. 14, 18. Juli 2003 (2003-07-18), Seiten 2053-2058, XP004435220

## Beschreibung

Die Erfindung betrifft ein Verfahren zur Herstellung chiraler, nicht-racemischer, cis-konfigurierter Cyclohexanole bzw. Cyclohexanol-Derivate der Formel (I). 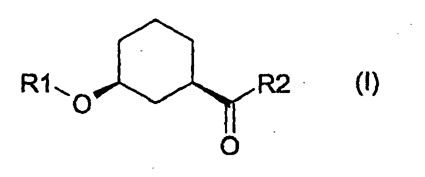 Cis-konfigurierte Hydroxycyclohexancarbonsäure-Derivate der Formel (I) sind zentrale Bausteine bzw. unmittelbare Vorstufen für die in der DE-Anmeldun Nr. 103 08 355.3 oder WO2004/076426 beschriebenen Arzneimittelwirkstoffe, die eine therapeutische Modulation des Lipid- und/oder Kohlenhydratstoffwechsels erlauben und sich somit zur Prävention und/oder Behandlung von Typ II Diabetes und Artherosklerose eignen.

Die in den oben genannten Patentanmeldungen beschriebenen Synthesen der nichtracemischen, cis-konfigurierten Cyclohexanol-Bausteine bzw. -Derivate kommen als technische Verfahren nicht in Betracht: Die Trennung der Isomeren, z. B. die Trennung der Enantiomeren (Racematspaltung) per Chromatographie an chiraler Phase, ist zu aufwendig und zu teuer. Darüber hinaus ist es für chromatographische Enantiomerentrennungen erforderlich, dass die racemische Verbindung in guter chemischer Reinheit vorliegt, was sich vielfach nur durch eine zusätzliche, vorgeschaltete Chromatographie erreichen lässt.
Darüber hinaus sind zahlreihe Reaktionen im technischen Maßstab nicht durchführbar. Dazu zählen vor allem Alkylierungen mit NaH in DMF, die bekanntermaßen ein hohes Sicherheitsrisiko darstellen (C&EN, September 13, 1982,5).

Die darüber hinaus in der Literatur bekannten Methoden zur Synthese optisch reiner, cis-konfigurierter 3-Hydroxy-cyclohexancarbonsäure-Derivate kommen für die Herstellung größerer Mengen der zentralen Bausteine der oben genannten Arzneimittelwirkstoffe nicht in Frage bzw. sind für die Entwicklung eines technischen Prozesses nicht geeignet, da Stufenzahl und/oder Ausbeute, Aufwand für Reinigungen, insbesondere für cis/trans-Trennungen, und vielfach auch die optische Reinheit unakzeptabel sind.
Zum Beispiel: Einige der in der Literatur beschriebenen synthetische Methoden zur Herstellung von optisch reinen, cis-konfigurierten 3-Hydroxy-cyclohexancarbonsäure-Derivaten basieren auf der Hydrierung bzw. Dearomatisierung von m-Hydroxybenzoesäure bzw. ihren Derivaten und anschließender klassischer Racematspaltung durch Salzbildung. Im Falle der Hydrierung von m-Hydroxybenzoesäure in Gegenwart von PtO₂ in Ethanol (D. S. Noyce, D. B. Denney, J. Am. Chem. Soc. 1952, 74, 5912; vgl. J. A. Hirsch, V. C. Truc, J.Org. Chem. 1986, 51, 2218) ist beschrieben, daß sechs Kristallisationen erforderlich sind, um racemische cis-3-Hydroxycyclohexancarbonsäure chemisch rein in 13.8 % Ausbeute zu erhalten. Noyce und Denney beschreiben auch die Racematspaltung von cis-3-Hydroxycyclohexancarbonsäure mit Hilfe von Chinin-Trihydrat (Quinine trihydrate). Ausgehend von 500 g Chinin-Trihydrat und 188.3 g (R,S)-cis-3-Hydroxycyclohexancarbonsäure werden nach mehreren Kriställisationsschritten 162 g des (+)-cis-3-Hydroxycyclohexancarbonsäure-Chinin-Salzes erhalten. Exakte Angaben zur Höhe der optischen Reinheit (ee) werden nicht gemacht, Drehwerte werden angegeben. Eine weitere Methode zur Herstellung optisch aktiver cis-3-Hydroxycyclohexancarbonsäure nutzt die Fällung der racemischen cis-konfigurierten Carbonsäure mit Cinchonidin [a) D. S. Noyce, D. B. Denney, J. Am. Chem. Soc. 1952, 74, 5912; b) M. Nakazaki, K. Naemura, S. Nakahara, J.Org. Chem. 1979, 44, 2438] und anschließende Umkristallisationen des resultierenden Salzes aus Methanol bzw. Ethanol.

Die beschriebenen Methoden kommen für die Herstellung größere Mengen optisch aktiver cis-3-Hydroxycyclohexancarbonsäure-Derivate nicht in Frage, da die große Zahl chemischer Stufen bzw. Reinigungsschritte, die Verwendung große Mengen optisch reiner, chiraler Hilfsstoffe für die Racematspaltung, die unumgängliche Freisetzung des gewünschten Stereoisomeren aus dem Salz und nicht zuletzt die schlechten Gesamtausbeuten unpraktikabel und unökonomisch sind.

Die Herstellung von cis-3-Acetoxy-cyclohexan-carbonsäure-methylester, jedoch in racemischer Form, ist beschrieben in D. S. Noyce, H.I. Weingarten, J. Am. Chem. Soc. 1957, 70, 3098.

In einer jüngeren Arbeit (C. Exl, E. Ferstl, H. Hönig, R. Rogi-Kohlenprath, Chirality 1995, 7, 211) werden die Rh-katalysierten Hydrierungen von 3-Acetoxy-benzoesäure-(-)-menthylester und 4-Hydroxy-benzoesäure-(-)-menthylester in Methanol/Essigsäure bei 100 bar und 40 °C bzw. 35 °C beschrieben. In beiden Reaktionen werden mindestens vier Produkte gebildet, unter anderem auch das gewünschte Produkt. Die wesentlichen Nachteile der Reaktionen sind: a) eine hohe Stufenzahl durch die Herstellung der optisch aktiven Benzoesäureester und die spätere Abspaltung des chiralen Auxiliars; b) technisch anspruchsvolle Reaktionsbedingungen (100 bar); c) unbefriedigende Ausbeuten und hoher Reinigungsaufwand durch eine große Zahl von Nebenprodukten und d) niedrige optische Reinheiten. Abschließend kommen die Autoren selbst zu dem Urteil, daß ihr Verfahren nur von geringem praktischen Wert ist.

D. A. Evans, G. C. Fu und A. H. Hoveyda (J. Am. Chem. Soc. 1992, 114, 6671) beschreiben die Ir(I)-katalysierte Hydroborierung von sekundären bzw. tertiären Amiden der 3-Cyclohexencarbonsäure. Obwohl die Ausbeuten und die Diastereoselektion gut sind, besteht das Problem der Abtrennung des trans-Isomeren. Darüber hinaus erfordert die Überführung der genannten Amide in die gewünschten Verbindungen der Formel (I) entweder die Spaltung der Amidbindung unter relativ drastischen Bedingungen, was von partieller Epimerisierung und Lactonisierung begleitet ist, oder aber den direkten Umbau der Amide in die gewünschten Verbindungen der Formel (I), was z. B. den stereoselektiven Aufbau von Aminosäureresten bedeutet und vielstufig und damit unökonomisch ist. Als technisches Verfahren kommt dies nicht in Betracht.

Die Synthese von (1R,3R)-3-Hydroxy-4-cyclohexencarbonsäuremethylester, der eine direkte Vorstufe von (1R,3S)-3-Hydroxy-cyclohexancarbonsäuremethylester darstellt, ist beschrieben im 500 mg-Maßstab (J. A. Marshall, S. Xie, J. Org. Chem. 1995, 60, 7230 und zit. Lit). Schlüsselschritte der Synthese ist eine asymmetrische [4+2]-Cycloaddition zwischen einem optisch aktiven Bis-Acrylat und Butadien in Gegenwart von TiCl₄. Nach Abspaltung des chiralen Auxiliars erhält man (R)-3-Cyclohexencarbonsäure mit 95 % ee, die über lodlactonisierung und anschließende HI-Eliminierung in (1R,5R)-7-Oxabicyclo[3.2.1]oct-2-en-6-on überführt wird. Öffnung des ungesättigten Lactons mit NaHCO₃/MeOH ergibt (1R,3R)-3-Hydroxy-4-cyclohexencarbonsäuremethylester.
Die Synthese von (R)- oder (S)-3-Cyclohexencarbonsäure über derartige Cycloadditionen ist mehrfach beschrieben. Die zahlreichen Beispiele unterscheiden sich im wesentlichen durch den verwendeten chiralen Hilfsstoff. Beispielhaft seien noch einige Arbeiten genannt: a) W. Oppolzer, C. Chapuis, D. Dupuis, M. Guo, Helv. Chim. Acta 1985, 68, 2100; b) C. Thom, P. Kocienski, K. Jarowicki, Synthesis 1993, 475; c) B. M. Trost, Y. Kondo, Tetrahedron Lett. 1991, 32, 1613. Im technischen Maßstab erfordern diese Reaktionen besondere Maßnahmen um die sichere Handhabung der Acrylate und des Butadiens zu gewährleisten.
Gravierender Nachteil dieser Synthesen sind die großen Mengen an Iod und Kaliumiodid, die zur Lactonisierung der Cyclohexencarbonsäure verwendet werden: Zur Herstellung von 460 mg (1R,3R)-3-Hydroxy-4-cyclohexencarbonsäuremethyl-ester durch intramolekulare Cyclisierung von 760 mg Cyclohexencarbonsäure werden in der Arbeit von J. A. Marshall und S. Xie 1.61 g Iod (ca. 1 eq.) und 6.0 g (ca. 6 eq.) Kaliumiodid benötigt. In der Arbeit von A. S. Raw und E. B. Jang, (Tetrahedron 2000, 56, 3285) ist die Durchführung der Iodlactonisierungen sogar mit der dreifachen Menge Iod beschrieben: Aufgrund von Sicherheitsüberlegungen und aus ökologischer Sicht kommt die Durchführung einer solchen Reaktion im multi-kg-Maßstab nicht in Betracht. Die Herstellung der genannten Lactone erfordert darüber hinaus chromatographische Reinigungen.
Phenylseleno- und Phenylsulfenollactonisierung [a) K. C. Nicolaou, S. P. Seitz, W. J. Sipio, J. F. Blount, J. Am. Chem. Soc. 1979, 101, 3884; b) K. C. Nicolaou, Tetrahedron 1981, 37, 4097] stellen keine geeignete Alternative dar. Die verwendeten Reagenzien bzw. die entstehenden Produkte und Nebenprodukte sind nicht nur stark geruchsbelästigend, sondern vielfach auch toxisch und verursachen ökologische Schäden. Zur Abtrennung unerwünschter Se- bzw- S-Folge- bzw. -Nebenprodukte sind meist chromatographische Reinigungen erforderlich. Die Durchführung einer solchen Reaktion im multi-kg-Maßstab kommt somit nicht in Betracht.
Auch die Bromolactonisierung (C. Iwata, A. Tanaka, H. Mizuno, K. Miyashita, Heterocycles 1990, 31, 987 und dort zit. Lit.) stellt keine Alternative für den technischen Maßstab dar, da Bromide bzw. Bromquellen aus ökologischen Gründen vermieden werden sollten bzw. besondere Vorkehrungen erfordern.

(R)-Cyclohexencarbonsäure ist weiterhin zugänglich über enzymatische Desymmetrisierung von 1,2-Cyclohex-4-en-dicarbonsäureester (P. Kocienski, M. Stocks, D. Donald, M. Perry, Synlett 1990, 38), aber auch hier gelten die oben geschilderten Nachteile der Iodlactonisierung.

Eine weitere Alternative besteht darin 2-Iodo-7-oxabicyclo[3.2.1]octan-6-on (auch 4-Iodo-6-oxabicyclo[3.2.1]octan-7-on genannt), das unmittelbare Produkt der Iodlactonisieung von Cyclohexencarbonsäure, mit Bu₃SnH zum gesättigten Lacton zu reduzieren, das dann z. B. mit NaOEt in Ethanol in 3-Hydroxycyclohexancarbonsäureethylester überführt werden kann. Die Entfernung des Iods ist in zahlreichen Beispielen beschrieben, u. a. in A. S. Raw, E. B. Jang, Tetrahedron 2000, 56, 3285. Die Aufarbeitung der Bu₃SnH-Reaktion und die vollständige Entfernung der resultierenden Sn- und Iod-Verbindungen ist häufig schwierig, erfordert meist zusätzliche chromatographische Reinigungen und ist somit für einen technischen Prozesse ebenfalls nicht wünschenswert. Gleiches gilt für die Entfernung der Se-, S- und Br-Verbindungen im Falle der Phenylseleno-, der Phenylsulfeno- und der Bromolactonisierung.

Die enzymatische Racematspaltung des cis-3-Hydroxycyclohexancarbonsäuremethylesters bzw. des Tetrahydropyranylderivates durch α-Chymotrypsin-katalysierte Esterhydrolyse (J. B. Jones, P. W. Marr, Tetrahedron Lett. 1973, 3165) ist ebenfalls kein geeignetes Verfahren, da die optischen Reinheiten der Produkte unbefriedigend sind: Die durchgeführten Experimente führten zu 42 % bzw. 50 % ee. Durch eine optimierte Kontrolle und Steuerung des Umsatzes lassen sich Enantiomerenüberschüsse von 85 % erreichen. Dies ist einerseits unzureichend und andererseits auch nur möglich, wenn man deutliche Einbußen bei den Ausbeuten in Kauf nimmt.

Die Herstellung von 3-Oxo-cyclohexen-1-carbonsäuremethylester bzw. 3-Oxo-cyclohexan-carbonsäuremethylester durch Reduktion von m-Methoxybenzoesäure mit Natrium in flüssigem Ammoniak ist beschrieben in M. E. C. Biffin, A. G. Moritz, D. B. Paul, Aust. J. Chem. 1972, 25, 1320.

Die stereoselektive mikrobielle Reduktion racemischer 3-Oxo-cyclohexancarbonsäureester mit Rhizopus arrhizus und anschließendeTrennung der Diastereomeren führt ebenfalls zu optisch aktiven cis-3-Hydroxycyclohexancarbonsäureestern (F. Trigalo, D. Buisson, R. Azerad, Tetrahedron Lett., 1988, 29, 6109 und zit. Lit.). Die aufwendige Trennung der Diastereomeren ist für eine Übertragung in den technischen Maßstab unattraktiv.

Die Reduktion von (R)-3-Oxo-cyclohexan-carbonsäuremethylester mit HLAD (horse liver alcohol dehydrogenase) in Gegenwart von NADH führt zum cis/trans-Gemisch des 3-Hydroxycyclohexancarbonsäuremethylesters (J. J. Willaert, G. L. Lemiere, L. A. Joris, J. A. Lepoivre, F. C. Alderweireldt, Bioorganic Chemistry 1988, 16, 223). Auch diese Methode ist daher zur Herstellung optisch reiner cis-3-Hydroxycyclohexancarbonsäure-Bausteine ungeeignet.

Die asymmetrische Reduktion von 3-Oxo-cyclohexen-1-carbonsäureisopropylester mit Geotrichum candidum (L.Fonteneau, S. Rosa, D. Buisson, Tetrahydron: Asymmetry 2002, 13, 579) ist ebenfalls nicht von Interesse, da nur das trans-Isomere des 3-Hydroxycyclohexancarbonsäureisopropylesters gebildet wird.

Die Untersuchung der Reaktion von Shikimat-Dehydrogenase mit 3-Oxo-cyclohexancarbonsäure in Gegenwart von NaDPH zeigt, daß dieses Enzym das (S)-Enantiomere mit 90 % Ausbeute in die entsprechende trans-3-Hydroxycyclo-hexancarbonsäure überführt (T. D. H. Bugg, C. Abell, J. R. Coggins, Tetrahedron Lett. 1988, 29, 6779). Diese Reaktion ist somit ebenfalls ungeeignet.

Auch die Biosynthese von Cyclohexancarbonsäure in Alicyclobacilus acidocaldarius (früher Bacillus acidocaldarius) und Streptomyces collinus, die ausgehend von Shikimisäure über 3-Hydroxy-cyclohexancarbonsäure verläuft, kann technisch nicht genutzt werden, da es sich bei der 3-Hydroxy-cyclohexancarbonsäure um das transkonfigurierte (1S,3S)-Isomere handelt (B. S. Moore, K. Poralla, H. G. Floss, J. Am. Chem. Soc. 1993, 115, 5267).

Ziel der vorliegenden Erfindung war es daher, ein Verfahren zu entwickeln, dass die genannten Nachteile nicht aufweist.

Gegenstand der vorliegenden Erfindung ist ein Verfahren zur Herstellung chiraler, nicht racemischer Verbindungen der Formeln (la) und (Ib) 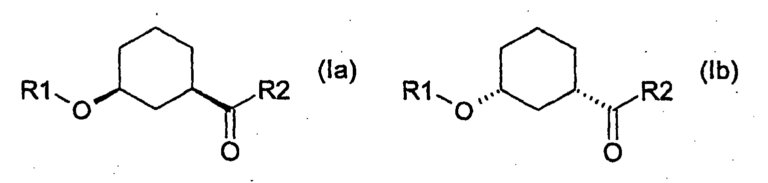 mit:
- R¹: 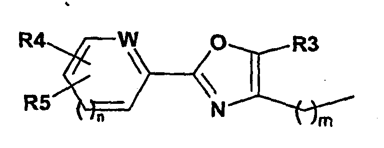 worin bedeuten:
- R³: H, (C1-C6)-Alkyl, (C3-C8)-Cycloalkyl, (C1-C3)-Alkyl-(C3-C8)-Cycloalkyl, Phenyl, (C1-C3)-Alkyl-Phenyl, (C5-C6)-Heteroaryl, (C1-C3)-Alkyl-(C5-C6)-Heteroaryl oder (C1-C3)-Alkyl, das durch F ganz oder teilweise substituiert ist;
- R⁴, R⁵: unabhängig voneinander H, F, Cl, Br, CF₃, OCF₃, (C1-C6)-Alkyl, O-(C1-C6)-Alkyl, SCF₃, SF₅, OCF₂-CHF₂, (C6-C10)-Aryl, (C6-C10)-Aryloxy, OH, NO₂; oder
- R⁴ und R⁵: zusammen genommen mit den sie tragenden C-Atomen einen kondensierten, teilweise oder nicht gesättigten bicyclischen (C6-C10)-Aryl- oder (C5-C11)-Heteroaryl-Ring bilden;
- W: CH, N, falls n = 1;
- W: O, S, NR⁶, falls n = 0;
- m: 1-6;
- R⁶: H, (C1-C6)-Alkyl-Phenyl, (C1-C6)-Alkyl;
oder
- R¹: eine OH-Schutzgruppe (SG), wie z.B. Benzyloxymethy, Benzyl, para-Methoxybenzyl, tert.-Butyldimethylsilyl (TBDMS), tert.-Butyldiphenylsilyl (TBDPS), Tetrahydropyranyl (THP), 1-Ethoxyethyl (EE), 1-ethyl-1-methoxyethyl oder 1-Methyl-1-benzyloxyethyl;
und
- R²: 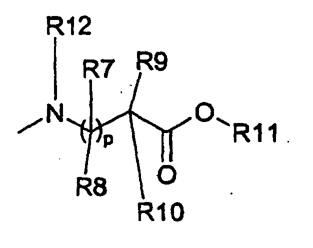 worin bedeuten:
p 0-2;
R⁷ H, (C1-C6)-Alkyl;
R⁸ H, (C1-C6)Alkyl;
R⁹ H, F, (C1-C6)-Alkyl;
R¹⁰ H, F, (C1-C6)-Alkyl, O-(C1-C6)-Alkyl, (C2-C6)-Alkenyl, (C2-C6)-Alkinyl, (C3-C8)-Cycloalkyl, Phenyl, wobei Alkyl, Alkenyl, Alkinyl und Cycloalkyl gegebenenfalls substituiert sein kann durch einen oder mehrere Reste aus der Reihe Hydroxy, Phenyl, (C5-C11)-Heteroaryl, O-(C1-C6)Alkyl und NR¹³R¹⁴, und Phenyl gegebenfalls substituiert sein kann durch einen oder mehrere Reste aus der Reihe Hydroxy, O-(C1-C6)-Alkyl, F und CF₃, mit der Maßgabe, dass R¹⁰ ungleich NR¹³R¹⁴ oder O-(C1-C6)-Alkyl, falls R⁹ = F ist;
R⁹ und R¹⁰ zusammen mit dem sie tragenden C-Atom (C3-C8)-Cycloalkyl;
R¹⁰ und R¹² zusammen Pyrrolidin oder Piperidin, falls p = 0 ;
R¹¹ H, (C1-C8)-Alkyl, Benzyl, (C1-C4)Alkyl-(C6-C10)-Aryl, (C1-C4)-Alkyl-O-(C1-C4)-Alkyl, Phenyl-(C1-C4)-Alkyl, wobei Alkyl, Benzyl, Phenyl, Aryl, gegebenenfalls ein- oder mehrfach substituiert sein können durch O-(C1-C6)-Alkyl, OCH₂CH₂-OMe, F, Cl, Br, I, Si(CH₃)₃, OSi(CH₃)₃, Si(iPr)₃, OSi(iPr)₃, OCH₂CH₂-SiMe₃, OCH₂-Si(iPr)₃, O-CH₂-C₆H₅, SO₂C₆H₄-p-Me, SMe, CN, NO₂, CH₂COC₆H₅;
R¹² H, (C1-C6)-Alkyl, (C2-C6)-Alkenyl, (C2-C6)-Alkinyl, Benzyl, CO-(C1-C6)-Alkyl, CO-Phenyl, C(O)-O-(C1-C6)-Alkyl, Allyloxycarbonyl (ALOC), Benzyloxycarbonyl (Cbz, Z), 9-Fluorenylmethyloxycarbonyl (FMOC), (C1-C4)-Alkyl-(C6-C10)-Aryl, (C1-C4)-Alkyl-(C5-C11)-Heteroaryl, (C1-C4)-Alkyl-O-(C1-C4)-Alkyl, Phenyl-(C1-C4)-Alkyl, (C5-C6)-Heteroaryl-(C1-C4)-Alkyl; SO2-(C1-C6)-Alkyl, SO2-(C1-C6)-Alkyl-SO₂-(C1-C6)-alkyl, SO₂-Phenyl, wobei Phenyl gegebenenfalls substituiert sein kann durch (C1-C6)-Alkyl, O-(C1-C6)-Alkyl, F, Cl;
R¹³ (C1-C6)-Alkyl;
R¹⁴ (C1-C6)-Alkyl-Phenyl, (C1-C6)-Alkyl;
dadurch gekennzeichnet, dass man
A)
   a) Lactonöffnung (LO)
      racemisches 6-Oxabicyclo[3.2.1]octan-7-on der Formel (II) 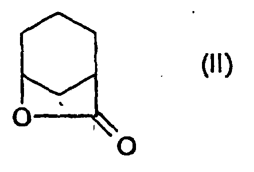 mit einer Verbindung der Formel (III)

      HO-R¹⁵ (III)

      mit
      - R¹⁵: H, (C1-C8)-Alkyl, (C3-C8)-Cycloalkyl (C2-C8)-Alkenyl, (C2-C8)-Alkinyl, Benzyl, (C1-C4)-Alkyl-(C6-C10)-Aryl, (C1-C4)-Alkyl-(C5-C11)-Heteroaryl, (C1-C4)-Alkyl-O-(C1-C4)-Alkyl, Phenyl-(C1-C4)-Alkyl, (C5-C6)-Heteroaryl-(C1-C4)-Alkyl, wobei Alkyl, Benzyl, Phenyl, Aryl, Heteroaryl gegebenenfalls ein- oder mehrfach substituiert sein können durch Phenyl, O-(C1-C6)-Alkyl, OCH₂CH₂-OMe, OTs, F, Cl, Br, I, Si(CH₃)₃, OSi(CH₃)₃, Si(iPr)₃, OSi(iPr)₃, OCH₂CH₂-SiMe₃, OCH₂-Si(iPr)₃, OTHP, O-CH₂-C₆H₅, SO₂C₆H₄-p-Me, SMe, CN, NO₂, COOH, CONH₂, CH₂COC₆H₅, CO-Benzoxy, CO-O(C1-C6)-Alkyl, NHTs, NHAc, NHBoc, NHAloc, NHBenzyl;
      in Gegenwart von geeigneten Basen oder Säuren in einem geeigneten Lösungsmittel umsetzt (z. B. mit Hydroxiden wie NaOH oder CsOH in Wasser oder z. B. mit Carbonaten wie K₂CO₃ in Alkoholen oder z. B. mit Säuren wie HCl in Wasser) oder mit Säurehalogeniden wie Acetylchlorid in Alkoholen, bevorzugt Acetylchlorid in Isopropanol, wobei im Falle der wäßrigen Reaktionen die Bedingungen der Aufarbeitung darüber entscheiden, ob man z.B. das Cs- (z. B. mit CsOH) oder das Na-(z. B. mit NaOH) oder das Ammonium-Salz (z. B. mit NH₃), oder aber die freie Säure erhält,
      z. B. zu einer racemischen cis-konfigurierten Verbindung der Formel (IV), 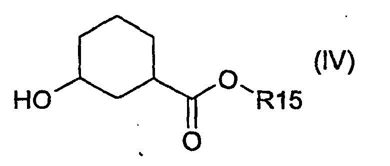 worin R¹⁵ wie oben definiert ist oder zu einer Verbindung der Formel (IV), die je nach Aufarbeitung in ionischer Form, z.B. als Cs⁺-, Li⁺-, K⁺-, NH₄⁺-, Ca²⁺-, Ba²⁺-, Mg²⁺- Salz vorliegen kann und worin R¹⁵ auch Cs, Li, K, NH₄, Ca, Ba, Mg bedeutet, und gegebenenfalls das so erhaltene Produkt, z. B. eine Verbindung der Formel (IV) mit R¹⁵ = Cs mit einem Alkylierungsmittel wie Benzylbromid zu einer Verbindung der Formel (IV) mit R¹⁵ = CH₂C₆H₅, d.h. einem anderen Produkt, worin R¹⁵ wie oben definiert ist, weiter umsetzt;
   b) Enzymatische Esterbildung (EB) + Trennung (T)
      die erhaltenen Verbindungen der Formel (IV) einer stereoselektiven enzymatischen Esterbildung (EB) unterwirft, wobei die Hydroxyverbindungen in einem organischen Lösungsmittel (wie z. B. Dichlormethan) mit einem Acyldonor (wie z.B. einem Vinylester R¹⁶-O-CH=CH₂. bevorzugt Vinylacetat, oder einem Säureanhydrid R¹⁶-O-R¹⁶ bevorzugt Bernsteinsäureanhydrid und Glutarsäureanhydrid), und dem Enzym aus der Gruppe der Hydrolasen versetzt und die resulltierende Mischung bei -20 bis 80 °C gerührt wird und nach Ablauf der Reaktion das eine Stereoisomere als Ester der Formel (Vb) 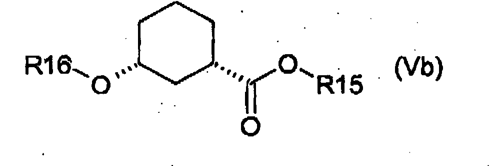 worin
      - R¹⁶: C(=O)-(C₁-C₁₆)-Alkyl, C(=O)-(C₂-C₁₆)-Alkenyl, C(=O)-(C₃-C₁₆)-Alkinyl, C(=O)-(C₃-C₁₆)-Cycloalkyl, wobei ein oder mehrere Kohlenstoffatome durch Sauerstoffatome ersetzt sein und substituiert sein können mit 1-3 Substituenten aus der Gruppe F, Cl, Br, CF₃, CN, NO₂, Hydroxy, Methoxy, Ethoxy, Phenyl, CO-O(C₁-C₄)-Alkyl und CO-O(C₂-C₄)-Alkenyl; wobei Phenyl, CO-O(C₁-C₄)-Alkyl und CO-O(C₂-C₄)-Alkenyl wiederum substituiert sein können mit 1-3 Substituenten aus der Gruppe F, Cl, Br, CF₃ bedeutet, und
      - R¹⁵: wie oben definiert ist,
      und das andere Stereoisomere unverändert als Alkohol der Formel (IVa) 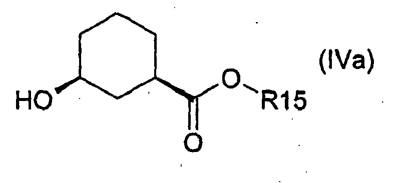 vorliegt, und daher durch Ausnutzung ihrer unterschiedlichen chemischen bzw. physikochemischen Eigenschaften (z. B. R_{f}-Werte oder Löslichkeitsunterschiede in Wasser oder anderen Lösungsmitteln) voneinander getrennt werden können (Trennung T), z. B. durch einfache Chromatographie an Kieselgel, durch Extraktion (z. B. Heptan / Methanol oder org. Lösungsmittel/Wasser) oder aber durch eine weiterführende chemische Folgereaktion z. B. der Hydroxyverbindung, an der der Ester nicht teilnimmt, wobei
      die als Alkohol anfallenden Enantiomere der Formel (IVa) wie unter d) beschrieben weiter verarbeitet werden, oder
   c) Esterspaltung (ESp)
      die als acylierte Verbindungen anfallenden Enantiomeren der Formel (Vb) zu chemisch enantiomeren Alkoholen (IVb) nach bekannten Verfahren verseift oder z. B. durch Umsetzung mit K₂CO₃ in Methanol zum optisch aktiven (1S,5R)-6-Oxabicyclo[3.2.1]octan-7-on intramolekular umestert, welches in eine isomere Form des Produktes überführt werden kann (siehe Schemata unten) 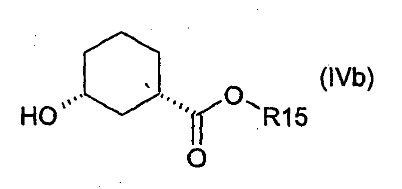 oder die Verbindung der Formel (Vb) z. B. durch lipasekatalysierte Spaltung beider Esterfunktionen zur optisch aktiven Verbindung der Formel (IVb, mit R¹⁵ = H) umwandelt, welche in eine isomere Form des Produktes überführt werden kann (siehe Schemata unten)
   d) Alkylierung (Alk-R¹ / Alk-SG)
      weiter mit Verbindungen der Formel (VI)

      R¹-X (VI)

      worin
      - R¹: 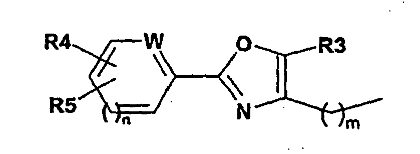 bedeutet und R³, R⁴, R⁵, W, n und m wie oben definiert sind, oder
      - R¹: für eine OH-Schutzgruppe (SG) steht wie oben definiert, mit Ausnahme von THP, EE, 1-Methyl-1-methoxyethyl oder 1-Methyl-1-benzyloxyethyl ;
      und
      - X: Cl, Br, I, OTs, OMs, OTf bedeutet;
      in Gegenwart von Basen in einem geeigneten Lösungsmittel zu den Verbindungen der Formeln (VIIa) oder (VIIb); 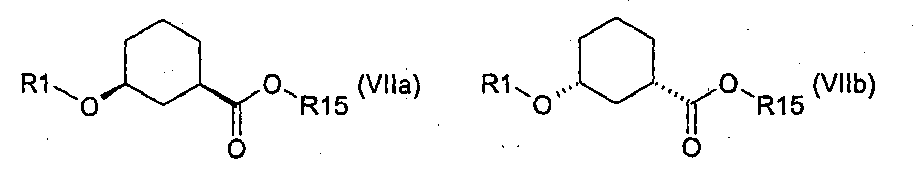 oder im Falle R¹ = SG zu den Verbindungen der Formeln (VIIIa) oder (VIIIb) 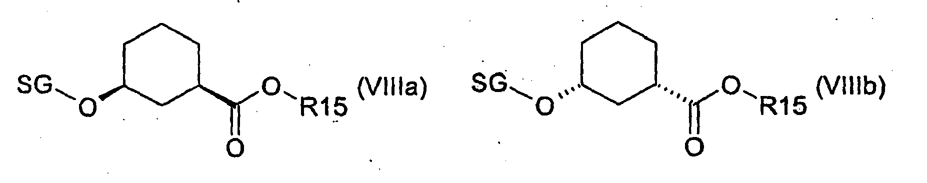 oder
      - R¹: für ein OH-Schutzgruppe (SG) steht wie Tetrahydropyranyl (THP), 1-Ethoxyethyl, 1-Methyl-1-methoxyethyl oder 1-Methyl-1-benzyloxyethyl steht;
      und
      zur Einführung der OH-Schutzgruppen die Verbindungen der Formel (IVa) und (IVb) säurekatalysiert mit den entsprechenden, bekannten Enolether ebenfalls zu Verbindungen der Formeln (VIIIa) oder (VIIIb) umgesetzt werden;
      und
   e) direkte Umsetzung oder Esterspaltung & Kupplung (dU o. Sp + K)
      e1) die erhaltenen Verbindungen der Formeln (VIIa) oder (VIIb) bzw. die Verbindungen der Formeln (VIIIa) oder (VIIIb) in einer direkten Umsetzung (dU), z. B. durch Reaktion eines Amins der Formel (IX)

         R²-H (IX)

         worin
         R² 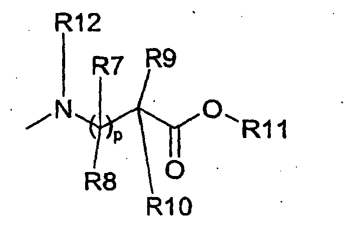 worin R7, R8, R9, R10, R11, R12 und p wie oben definiert sind,
         bzw. dem korrespondierenden Lithium- bzw- Dimethylaluminium-Derivat, oder durch Reaktion der Verbindungen der Formeln (VIIa) oder (VIIb) bzw. der Verbindungen der Formeln (VIIIa) oder (VIIIb) und dem Amin bzw. Aminosäurederivat R²-H der Formel (IX) in Gegenwart aktivierender Reagenzien bzw. Katalysatoren, wie beispielsweise dem Cyanid-Ion, in Verbindungen der Formeln (la) oder (Ib) oder deren isomere Formen überführt, 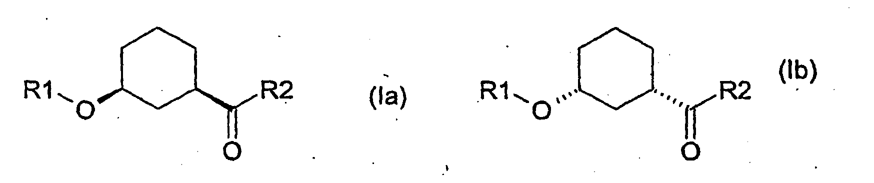 oder im Falle R¹ = SG in Verbindungen der Formeln (Xa) oder (Xb) überführt, 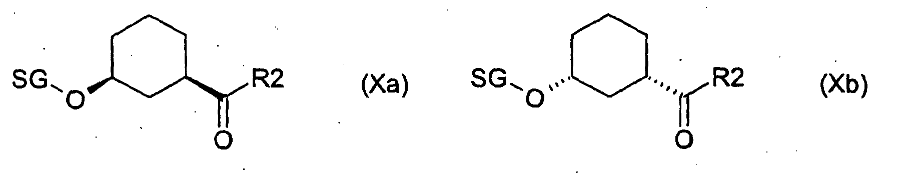 oder
      e2) die erhaltenen Verbindungen der Formeln (VIIa) oder (VIIb) bzw. (VIIIa) oder (VIIIb) einer Esterspaltung, z. B. einer basischen Hydrolyse mit wäßriger NaOH, einer sauren Hydrolyse mit wäßriger HCl, oder einer enzymatischen Hydrolyse mit einer Lipase, oder einer Hydrogenolyse mit H₂ in Gegenwart von Pd/C, und die so erhaltenen Verbindungen der Formeln (XIa) oder (XIb), bzw. (XIIIa) oder (XIIIb) 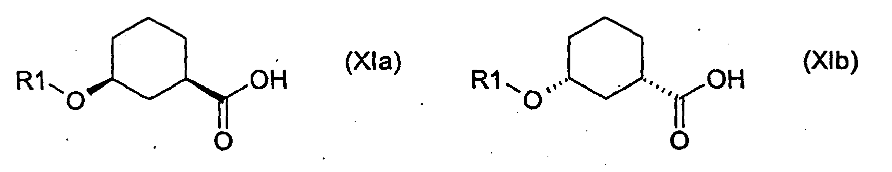 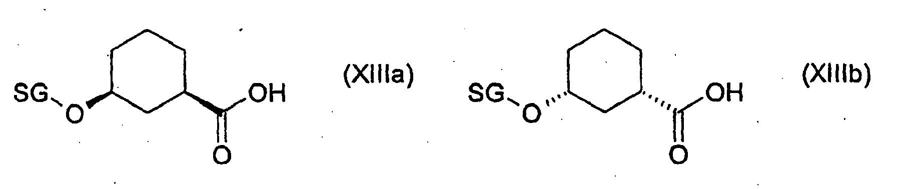 bzw. die entsprechenden Salze, z. B. dem Li-, Na-, K-, Cs- oder NH₄-Salz dieser Verbindungen, einer anschließender Kupplung unterwirft mit einer Verbindung der Formel (IX)

         R²-H (IX)

         worin
         R² 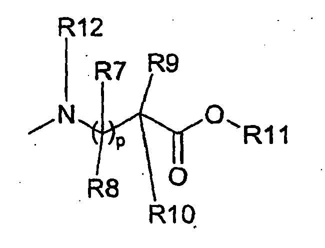 bedeutet und worin R7, R8, R9, R10, R11, R12 und p wie oben definiert sind,
         in Gegenwart von wasserentziehenden oder aktivierenden Reagenzien, wie z. B. PPA (Propanphosphonsäureanhydrid), TOTU ([Cyano(ethoxycarbonyl) methylenamino]-1,1,3,3-tetramethyluroniumtetrafluoroborat), EDC (1-(3-(dimethylamino)-propyl)-3-ethylcarbodiimid-Hydrochlorid), HOBt 1-Hydroxybenzotriazol), DMAP (4-Dimethylaminopyridin), DCC (Dicyclohexylcarbodiimid), CDI (N, N'-Carbonyldiimidazol) zu einer Verbindung der Formeln (la) oder (Ib) oder deren isomere Formen; 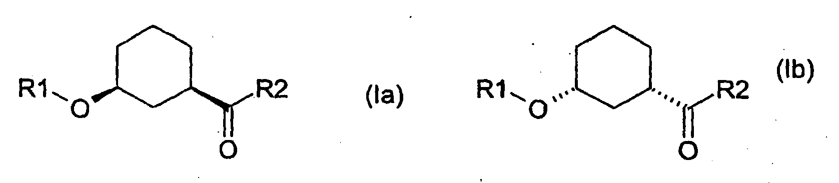 und gegebenenfalls
   f) Abspaltung der Schutzgruppe SG (AbSG)
      falls R¹ für eine OH-Schutzgruppe (SG) steht, wie oben unter R¹ definiert, werden die Verbindungen der Formeln (Xa) oder (Xb) 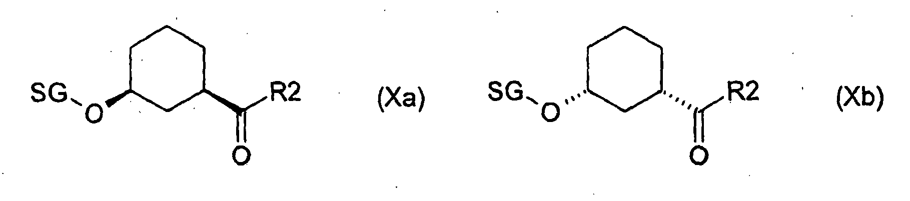
   worin R² und SG wie oben definiert sind,
   durch Abspaltung der Schutzgruppe nach bekannten Verfahren, wie z. B. Abspalten von SG = Benzyloxymethyl oder SG = Benzyl durch Hydrierung an Pd/C, oder Abspalten von SG = para-Methoxybenzyl mit beispielsweise DDQ (2,3-Dichloro-5,6-dicyanobenzoquinon), Abspalten von SG = tert.-Butyl-dimethylsilyl z. B. mit Bu₄NF, oder Abspalten von SG = Tetrahydropyranyl (THP), SG = 1-Ethoxyethyl, SG = 1-Methyl-1-methoxyethyl oder SG = 1-Methyl-1-benzyloxyethyl, z. B. säurekatalysiert mit p-Toluolsulfonsäure oder HCl;
   in Verbindungen der Formeln (XIIa) oder (XIIb) 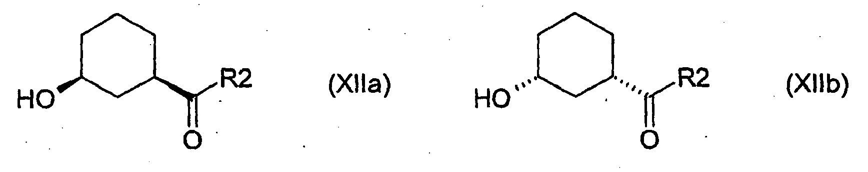 worin R² wie oben definiert ist, überführt, und dann entsprechend der angegebenen Verfahrensvarianten in die Verbindungen der Formeln (Ia) oder (Ib) oder deren isomere Formen umgewandelt;
   wobei es auch möglich ist die Reihenfolge der einzelnen Reaktionsschritte wie vorstehend unter A) beschrieben:
   A) LO → EB+T [→ ESp] → Alk-R¹ → dU oder Sp + K → Produkt/isomere Form
      zu ändern in:
   **B)** LO → EB+T [→ ESp] → dU oder Sp + K → Alk-R¹ → Produkt/ isomere Form
      oder
   **C)** LO → dU oder Sp + K → EB + T → [ESp] → Alk-R¹ → Produkt/ isomere Form
      oder
   **D)** LO → EB+T → [Esp] → Alk-SG → dU oder Sp + K → AbSG → Alk-R¹ → Produkt/ isomere Form,
      oder
   **E)** LO → Alk-SG → dU oder Sp + K → AbSG → ES + T → [ESp] → Alk-R¹ → Produkt/ isomere Form.

Nachfolgend sind mögliche Verfahrensvarianten in den Schemata I bis V dargestellt:

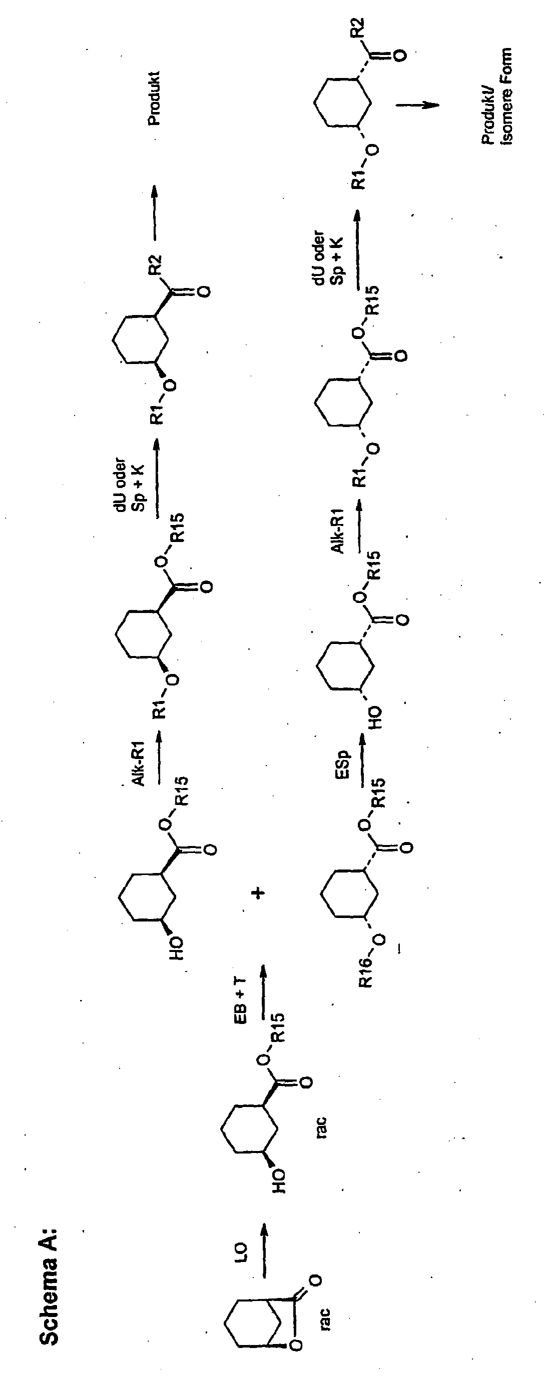

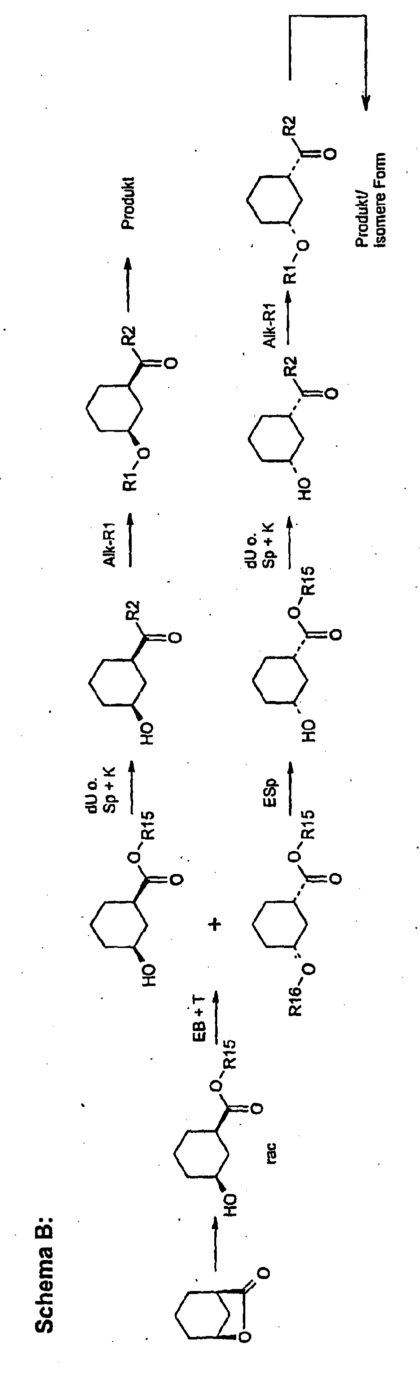

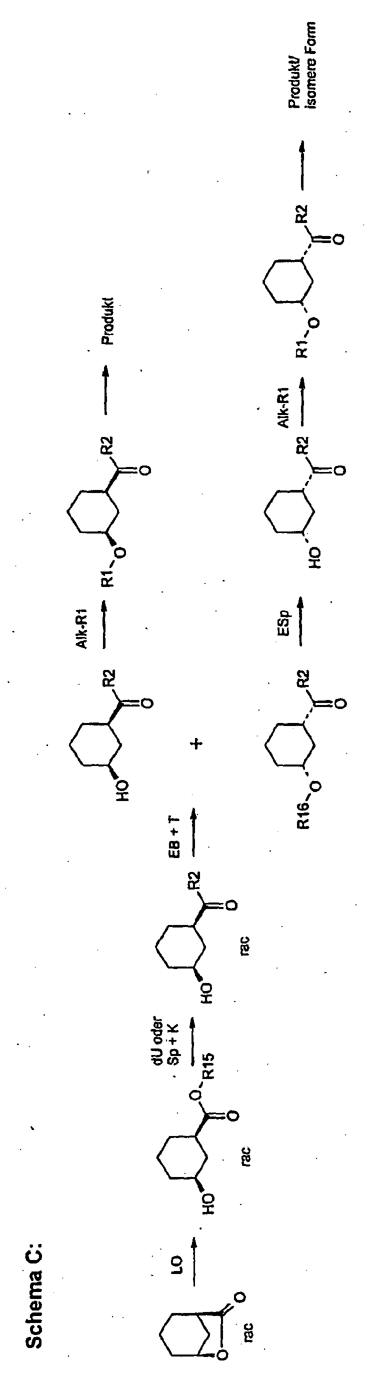

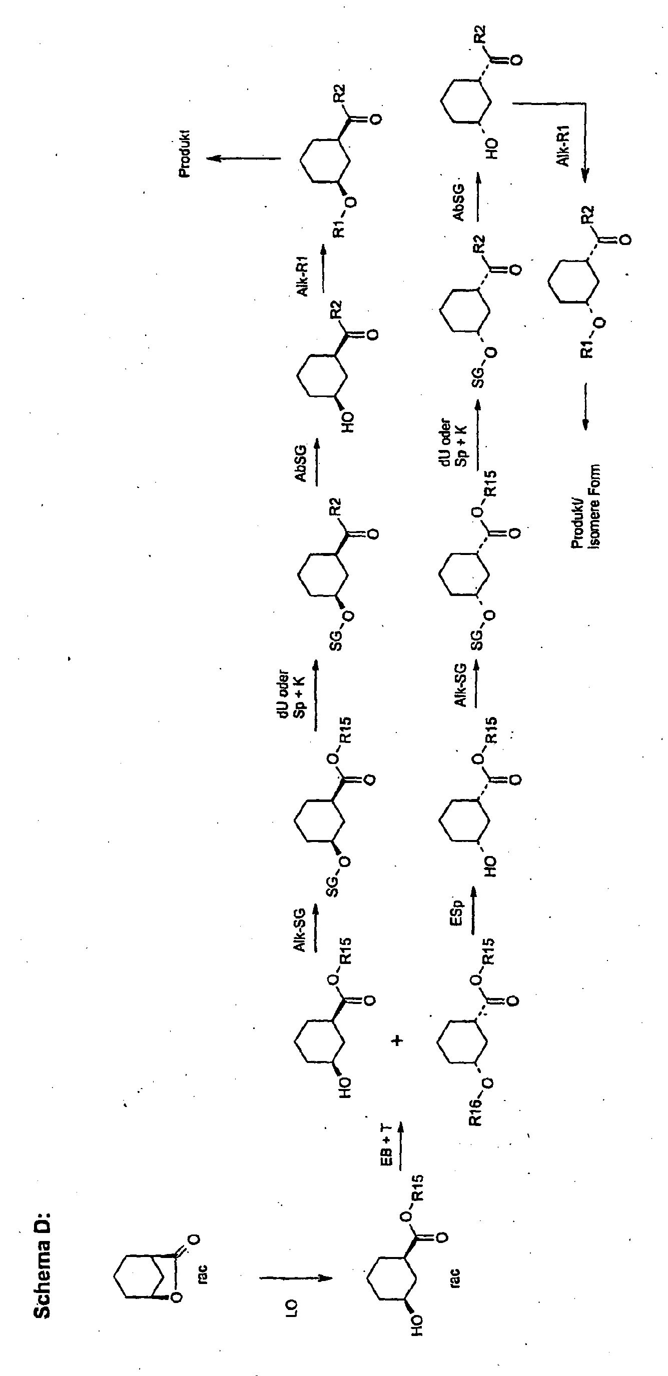

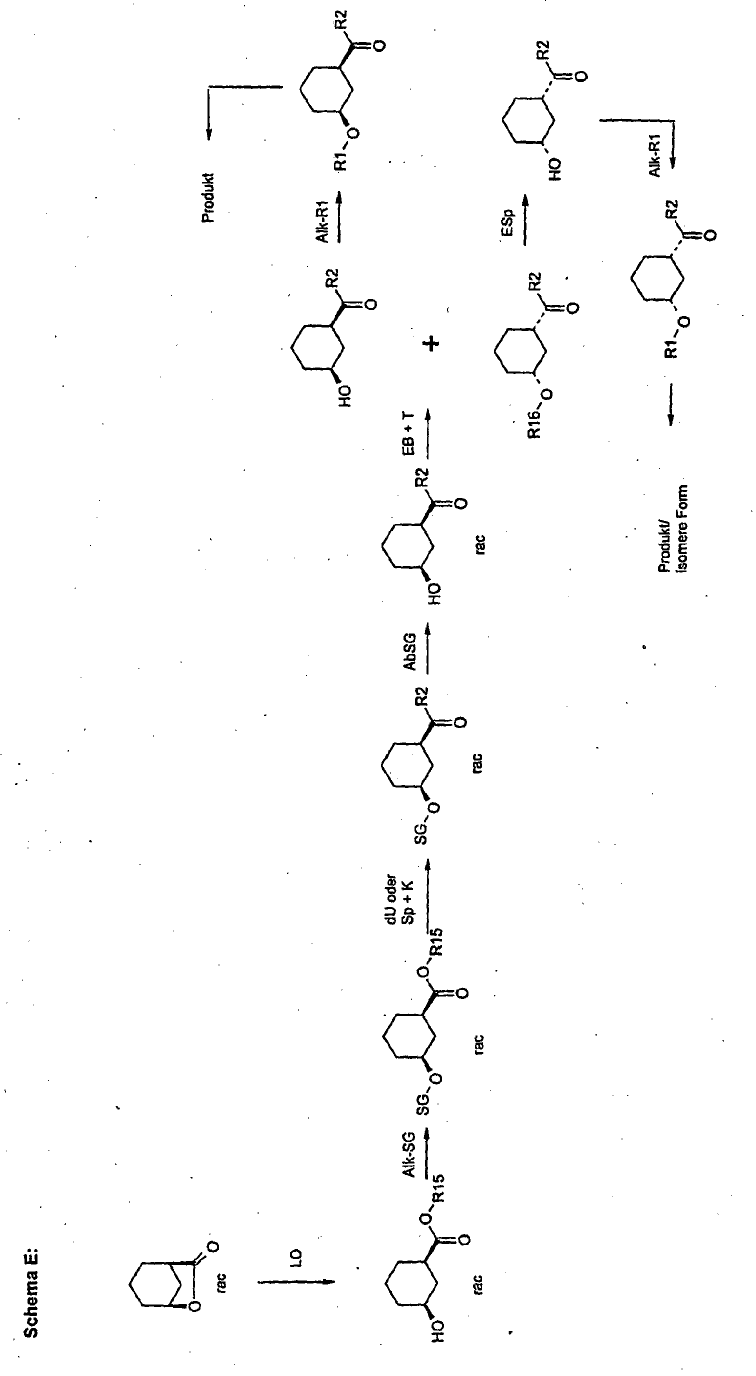

Die Verbindungen der Formeln (Ia) oder (Ib) haben zwei Asymmetriezentren am Cyclohexan-Ring. Hier ist die cis-Verknüpfung essentiell. Es ist jedoch auch möglich, dass weitere Asymmetriezentren auftreten, z.B. im Rest R2. Daher können die Verbindungen der Formeln (Ia) oder (Ib) in Form ihrer Racemate, racemischen Mischungen, reinem Enantiomere, Diastereomere und Disastereomer-Mischungen vorliegen. Die vorliegende Erfindung umfasst alle diese isomeren Formen der Verbindungen der Formeln (la) oder (Ib). Diese isomeren Formen können, wenn auch zum Teil nicht expressis verbis beschrieben, nach bekannten Methoden erhalten werden.

Unter einem heteroaromatischen Ring werden sowohl mono- als auch bi-cyclische Ringe verstanden mit maximal 4 Heteroatomen, insbesondere solche, die bis zu 4 Stickstoffatome und/oder 1 Sauerstoff bzw. 1 Schwefelatom enthalten, wie z.B.: Furan, Thiophen, Thiazol, Oxazol, Thiadiazol, Triazol, Pyridin, Triazin, Chinolin, Isochinolin, Indol, Benzothiophen, Benzofuran, Benzotriazol. Aromatische Ringe können mono- oder bicyclisch und auch anneliert sein, wie z.B. Naphthyl, Benzo[1,3]-dioxol, Dihydro-benzo[1,4]-dioxin.

Das erfindungsgemäße Verfahren ist ökonomisch, einfach und schnell. Es erfordert keine äquimolaren Mengen optisch reiner Ausgangs- oder Hilfsstoffe, keine teuren Reagenzien, keine Reagenzien etc., die ein besonderes Gefahrenpotential besitzen, keine Racematspaltung durch Chromatographie an chiralen Phasen, keine unverhältnismäßig großen Lösungsmittelmengen und keine kostenintensiven Arbeitsschritte.

Bevorzugt sind die vorstehend genannten Verfahren A), B) und D), besonders bevorzugt das Verfahren A).

Bevorzugt ist ein Verfahren zur Herstellung der Verbindungen der Formeln (Ia) und (Ib), worin ein oder mehrere Reste die folgenden Bedeutungen haben:
- R¹: 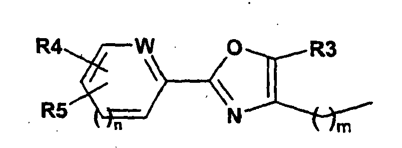 oder
- R¹: eine OH-Schutzgruppe (SG), wie z.B. Benzyloxymethy, Benzyl, para-Methoxybenzyl, tert.-Butyldimethylsilyl (TBDMS), tert.-Butyldiphenylsilyl (TBDPS), Tetrahydropyranyl (THP), 1-Ethoxyethyl (EE), 1-Methyl-1-methoxyethyl oder 1-Methyl-1-benzyloxyethyl;
mit
- R⁴: F, Br, CF₃, OCF₃, (C1-C6)-Alkyl, O-(C1-C6)-Alkyl, Phenyl; oder
solche, in denen der Substituent
- R⁴: in meta- oder in para-Stellung steht; oder
- R⁵: Wasserstoff ist; oder
- R⁴ und R⁵: zusammen mit dem Phenylring = Naphthyl; oder
- R³: H, (C1-C6)-Alkyl, (C3-C8)-Cycloalkyl, (C1-C3)-Alkyl-(C5-C6)-Cycloalkyl, Phenyl, (C1-C3)-Alkyl-Phenyl; oder
- W: CH, falls n = 1; oder
- m: 1; oder
- R²: 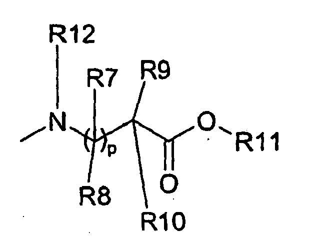 mit
- p: 0; oder
- R9: H, (C1-C6)-Alkyl; oder
- R9 und R10: zusammen mit dem sie tragenden C-Atom (C3-C6)-Cycloalkyl, besonders Cyclopentyl;
- R10: (C1-C6)-Alkyl, wobei Alkyl gegebenenfalls substituiert sein kann durch ein oder mehrere Reste aus der Reihe Hydroxy, Phenyl, (C5-C11)-Heteroaryl, (C1-C6)-Alkoxy und NR13R14, mit
- R13 und R14: H, (C1-C6)-Alkyl.
- R11: H, (C1-C8)-Alkyl, Benzyl, (C1-C4)-Alkyl-(C6-C10)-Aryl, wobei Alkyl, Benzyl, Aryl, gegebenenfalls ein- oder mehrfach substituiert sein können durch OMe, OCH₂CH₂-OMe, F, Cl, Br, Si(CH₃)₃, OSi(CH₃)₃, OCH₂CH₂-SiMe₃, O-CH₂-C₆H₅, , SMe, CN, NO₂, CH₂COC₆H₅;

Besonders bevorzugt ist ferner ein Verfahren zur Herstellung der Verbindungen der Formeln (Ia) und (Ib), worin bedeuten
- R10: (C1-C4)-Alkyl, (C1-C4)-Alkyl-O-(C1-C4)-Alkyl oder Benzyl;
- R11: H, (C1-C8)-Alkyl, Benzyl;
ganz besonders bevorzugt
- R10: (C1-C4)-Alkyl oder Benzyl,
- R11: H, (C1-C8)-Alkyl.

Ganz besonders bevorzugt ist auch ein Verfahren zur Herstellung der Verbindungen der Formeln (Ia) oder (Ib),
worin bedeuten
- R4: Br, CF₃, OCF₃, (C1-C6)-Alkyl, O-(C1-C6)-Alkyl;
- R5: H, (C1-C6)-Alkyl, O-(C1-C6)-Alkyl oder
- R4 und R5: zusammen mit dem Phenylring = Naphthyl;
- R3: CF₃, (C1-C6)-Alkyl, (C3-C6)-Cycloalkyl, Phenyl;
- W: CH, falls n = 1;
- m: 1;
- p: 0
- R9: H, (C1-C6)-Alkyl;
- R10: (C1-C6)-Alkyl, wobei Alkyl gegebenenfalls substituiert sein kann durch Phenyl;
- R10 und R12: zusammen mit den sie tragenden Atomen Pyrrolidin, falls p = 0;
- R9 und R10: zusammen mit dem sie tragenden C-Atom (C3-C6)-Cycloalkyl;
- R11: H,
- R12: H, (C1-C6)-Alkyl, Benzyl.

Die racemischen Verbindung der Formel (IV) werden durch Öffnung von 6-Oxabicyclo[3.2.1]octan-7-on mit Alkoholen oder in Gegenwart von Wasser hergestellt. Racemisches 6-Oxabicyclo[3.2.1]octan-7-on (II) ist kommerziell erhältlich und kann beispielsweise über Dearomatisierung, z. B. durch Hydrierung von m-Hydroxybenzoesäure bzw. -Derivaten und Cyclisierung der cis-3-Hydroxycyclohexancarbonsäure, synthetisiert werden.
Die Öffnung des Lactons kann, wie es in der Literatur für viele Lactone beschrieben ist (z. B. in M. Carballido, L. Castedo, C. Gonzalez, Tetrahedron Lett. 2001, 42, 3973), sowohl unter sauren als auch unter basischen Bedingungen erfolgen, z. B. mit Wasser in Gegenwart von Hydroxiden wie LiOH, z. B. mit Wasser in Gegenwart von Säuren wie Essigsäure, z. B. mit Alkoholen in Gegenwart von Basen wie K₂CO₃ und z. B. mit Alkoholen in Gegenwart von Säuren wie HCl.
Zur Öffnung des Lactons wird bevorzugt Acetylchlorid in Alkoholen verwendet.

Zur Racematspaltung der Hydroxyverbindungen werden diese in organischen Lösungsmitteln wie z. B. Dimethoxyethan (DME), Methyl-tert.-Butylether (MTBE), Diisopropylether (DIPE), THF, n-Hexan, Cyclohexan, Toluol, Chlorbenzol, Aceton, Dimethylformamid (DMF), Dichlormethan, 1,2-Dichlorethan und tert.-Butanol aufgenommen, Acyldonoren wie Vinylacetat, Vinylpropionat, Vinylbutyrat, 2,2,2-Trifluorethyl-2H,2H-perfluordecanoat, Ethoxyvinylacetat, p-Nitro- oder p-Chlorphenylacetat, Oximester, Acetanhydrid, Propionsäureanhydrid, Bernsteinsäureanhydrid, Glutarsäureanhydrid, iso-Valeriansäureanhydrid, 2,2,2-Trichlorethylbutyrat, 2,2,2-Trifluorethyl-2H,2H-perfluordecanoat werden zugesetzt und die Reaktionsmischung wird anschließend mit einem geeigneten Enzym aus der Gruppe der Hydrolasen versetzt und bei -20 bis 80 °C gerührt. Der Anteil an Cosolvens in der Lösung liegt vorzugsweise bei 10-90 %, ggf. ist es aber auch vorteilhaft die enzymatische Reaktion in reinem Acyldonor, z. B. Vinylacetat, ohne Cosolvens durchzuführen.

Nach Beendigung der Reaktion lassen sich die Produkte bzw. die Enantiomeren auf einfache Weise trennen, z. B. durch Extraktion nach literaturbekannten Methoden [a).T. Yamano, F. Kikumoto, S. Yamamoto, K. Miwa, M. Kawada,T. Ito, T. Ikemoto, K. Tomimatsu, Y. Mizuno, Chem. Lett. 2000, 48; b). B. Hungerhoff, H. Sonnenschein, F. Theil, J. Org. Chem. 2002, 67, 1781] oder durch Anwendung chromatographische Methoden.
Eine weitere Methode besteht darin, nach Ablauf der enzymatischen Reaktion die Wasserlöslichkeit der verbleibenden Hydroxyverbindung durch Derivatisierung, z. B. durch Acylierung mit cyclischen Anhydriden, wie z. B, mit Glutarsäureanhydrid, oder durch Überführung in einen Cholinester [a). H. Kunz, M. Buchholz, Chem. Ber. 1979, 112, 2145; b.) M. Schelhaas, S. Glomsda, M. Hänsler, H.-D. Jakubke, H. Waldmann, Angew. Chem. 1996, 108, 82] deutlich zu erhöhen und so eine Trennung von den wasserun- bzw. schlechtlöslichen Estern durch Extraktion zu erreichen. Nach der Trennung lässt sich die Derivatisierung der Alkohole durch chemische oder enzymatische Verseifung wieder rückgängig machen. Eine besonders interessante Möglichkeit zur Trennung der Enantiomeren besteht darin, im Falle der enzymatischen Acylierung den Acyldonor so zu wählen, dass das acylierte Enantiomere deutlich besser wasserlöslich ist als die nicht umgesetzte Hydroxyverbindung. Geeignete Acyldonoren sind beispielsweise cyclische Anhydride wie Bernsteinsäureanhydrid. Nach Ablauf der enzymatischen Acylierung trägt das Acylierungsprodukt eine freie Carboxylgruppe, die eine schnelle Abtrennung des Produktes durch wässrige Extraktion im Basischen, zum Beispiel mit gesättigter wäßriger NaHCO₃-Lösung, ermöglicht.

Als Enzyme werden Hydrolasen aus Säugetierlebern, wie z. B. Lipase aus Schweinepankreas (Fluka) oder aus Mikroorganismen, wie beispielsweise Lipase B aus Candida antarctica (Roche Diagnostics), Lipase A aus Candida antarctica (Roche Diagnostics). Lipase OF aus Candida rugosa (Meito Sangyo), Lipase SL aus Pseudomonas cepacia (Meito Sangyo), Lipase L-10 aus Alcaligenes spec. (Roche Diagnostics), Lipase QL aus Alcaligenes spec. (Meito Sangyo) und Glutaryl-7-ACA-Acylase (Roche Diagnostics) eingesetzt.
Bevorzugt ist Lipase B aus Candida antarctica (Roche Diagnostics), wobei es vorteilhaft sein kann, das freie Enzym oder eine immobilisierte Form des Enzyms, z. B. eines der drei zur Zeit kommerziell erhältlichen Produkte, zu verwenden.

Jedes der genannten Enzyme kann in freier oder in immobilisierter Form (Immobilized Biocatalysts, W. Hartmeier, Springer Verlag Berlin, 1988) eingesetzt werden. Die Enzymmenge wird in Abhängigkeit von der Reaktionsgeschwindigkeit bzw. von der angestrebten Reaktionszeit und von der Art des Enzyms (z. B. frei oder immobilisiert) frei gewählt und ist durch einfache Vorversuche leicht zu bestimmen. Die Wiedergewinnung des Enzyms kann durch Gefriertrocknung erfolgen. Die Abtrennung (und ggf. spätere Wiederverwendung) des Enzyms kann durch Immobilisierung erleichtert werden.

Durch geeignete Reaktionsführung gelingt es immer, zumindest ein Enantiomeres optisch rein zu erhalten. Strebt man optisch reinen Ester an, sollte der Umsatz im Falle der enzymatischen Esterbildung unter (oder gleich) 50 % sein. Strebt man optisch reinen Alkohol an, sollte der Umsatz im Falle einer enzym-katalysierten Esterbildung über (oder gleich) 50 % sein. Die Umsatzbestimmung der enzymatischen Reaktion erfolgte entweder per GC oder HPLC direkt aus der Reaktionsmischung oder durch Berechnung aus den optischen Reinheiten der Reaktionsprodukte (Ester und Säure), die ebenfalls direkt aus der Reaktionsmischung per GC bzw. HPLC an chiraler Phase bestimmt wurden.

Die Verbindungen R¹-X der Formel (VI) bzw. die korrespondierenden Alkohole R¹-OH, die als Vorstufen dienen können, sind kommerziell erhältlich oder können nach literaturbekannten Methoden hergestellt werden [a) The Chemistry of Heterocyclic Compounds (Ed.: A. Weissberger, E. C. Taylor): Oxazoles (Ed.: I. J. Turchi); b). Methoden der Organischen Chemie, Houben-Weyl 4. Auflage, Hetarene III, Teilband 1; c) O. Diels, D. Riley, Ber. Dtsch. Chem. Ges. 1915, 48, 897; d) W. Dilthey, J. Friedrichsen, J. Prakt. Chem. 1930, 127, 292; e) A. W. Allan, B. H. Walter, J. Chem. Soc. (C) 1986, 1397; f) P. M. Weintraub, J. Med. Chem. 1972, 15, 419; g) I. Simit, E. Chindris, Arch. Pharm. 1971, 304, 425; h) Y. Goto, M. Yamazaki, M. Hamana, Chem. Pharm. Bull. 1971, 19 (10), 2050-2057].

Die Verbindungen R¹-X der Formel (VI) werden in Gegenwart von Basen mit den optisch reinen, cis-konfigurierten 3-Hydroxy-cyclohexancarbonsäure-Derivaten umgesetzt. Geeignete Basen sind beispielsweise Hydroxide wie KOH, Carbonate wie Cs₂CO₃, Alkoholate wie KOtBu sowie Verbindungen wie LDA, BuLi, LiHMDS, KHMDS, NaH und NaHMDS. Geeignete Lösungsmittel sind beispielsweise THF, MTBE, DME, NMP, DMF, Toluol und Chlorbenzol.
Die Einführung der OH-Schutzgruppen (SG) durch Umsetzung der Verbindungen der Formel (IVa) und (IVb) mit einer Verbindung der Formel (VI) erfolgt nach literaturbekannten Methoden (T. W. Greene, P. G. Wuts, Protective Groups in Organic Synthesis, 3rd edition, John Wiley & Sons, Inc., 1999).
Darüber hinaus können zur Einführung von OH-Schutzgruppen wie Tetrahydropyranyl, 1-Ethoxyethyl, 1-Methyl-1-methoxyethyl oder 1-Methyl-1-benzyloxyethyl die Verbindungen der Formel (IVa) und (IVb) die entsprechenden, , bekannten Enolether (z. B. Dihydropyran und Ethylvinylether) nach literaturbekannten Methoden (T. W. Greene, P. G. Wuts, Protective Groups in Organic Synthesis, 3rd edition, John Wiley & Sons, Inc., 1999) verwendet werden.

R¹ wird als SG so gewählt wird, dass SG im Verlauf der späteren Synthese wieder leicht und selektiv abgespalten werden kann; SG ist also z.B. Benzyloxymethyl, Benzyl, para-Methoxybenzyl, tert.-Butyldimethylsilyl (TBDMS), tert.-Butyldiphenylsilyl (TBDPS), 1-Ethoxyethyl (EE), oder Tetrahydropyranyl (THP).
Die spätere Abspaltung der Schutzgruppe erfolgt ebenfalls nach bekannten Verfahren, wie z. B. Abspalten von SG = Benzyloxymethyl oder SG = Benzyl durch Hydrierung an Pd/C, oder Abspalten von SG = para-Methoxybenzyl mit beispielsweise DDQ (2,3-Dichloro-5,6-dicyanobenzoquinon), oder Abspalten von SG = tert.-Butyl-dimethylsilyl oder SG = tert.-Butyldiphenylsilyl z. B. mit Bu₄NF, oder Abspalten von SG = Tetrahydropyranyl (THP), SG = 1-Ethoxyethyl, SG = 1-Methyl-1-methoxyethyl oder SG = 1-Methyl-1-benzyloxyethyl, z. B. säurekatalysiert mit p-Toluolsulfonsäure oder HCl.

Die Amine bzw. Aminosäurederivate der Formel (IX) sind leicht zugänglich. Sowohl bei den Derivaten der proteinogen als auch der nicht-proteinogen Aminosäuren handelt es sich überwiegend um Bausteine, die aus der Peptidchemie bekannt und bei denen die verschiedenen isomeren als isomerenreine Verbindungen kommerziell erhältlich sind. Darüber hinaus können die verwendeten Aminosäurederivate der Formel (IX) mit Hilfe.literaturbekannter Methoden hergestellt werden [a) Houben-Weyl, Methoden er Organischen Chemie, 4. Auflage, Band E16d, Teilbände I u. II; b) C. Cativiela, M. D. Diaz-de-Villegas, Tetrahedron: Asymmetry 1998, 9, 3517; c) M. Beller, M. Eckert, Angew. Chem. 2000, 112, 1026].

Die direkte Umsetzung eines Esters der Formeln (IVa) oder (IVb), (VIIa) oder (VIIb) bzw. der Formeln (VIIIa) oder (VIIIb) mit einem Amin bzw. einem Aminosäurederivat der Formel (IX) können nach literaturbekannten Methoden [a) M. B. Smith, J. March, March's Advanced Organic Chemistry, 5th edition, John Wiley & Sons, Inc.,2001, S. 510 und dort zitierte Literatur; b) Literatur-Übersicht R. C. Larock, Comprehensive Organic Transformations, S. 987, VCH Publishers, Inc., 1989] erfolgen, z. B. über die korrespondierenden Lithium- bzw- Dimethylaluminium-Derivate, oder in Gegenwart geeigneter Katalysatoren, wie beispielsweise dem Cyanid-lon.

Die Durchführung der Esterspaltungen kann nach literaturbekannten Methoden (M. B. Smith, J. March, March's Advanced Organic Chemistry, 5th edition, John Wiley & Sons, Inc.,2001, S. 469 und dort zitierte Literatur) erfolgen, z. B. durch basischen Hydrolyse mit wäßriger NaOH, einer sauren Hydrolyse mit wäßriger HCl, oder einer enzymatischen Hydrolyse mit einer Lipase, oder z. B. im Falle eines Benzylesters durch Hydrogenolyse mit H₂ in Gegenwart von Pd/C.

Die Überführung der durch Esterspaltung gebildeten Cyclohexancarbonsäuren in die Verbindungen der Formeln (XIa) oder (XIb) erfolgt durch literaturbekannte Methoden der Amid- bzw. Peptid-Kupplung. Zur Knüpfung amidischer Bindungen stehen eine Fülle von Methoden zur Verfügung [a) Houben-Weyl, Methoden er Organischen Chemie, 4. Auflage, Band XV, Teilbände 1 u. 2; b) G. Benz in Comprehensive Organic Synthesis (Hrsg.: B. M. Trost), 1991, S. 381; c) Miklos Bodansky, Peptide Chemistry, 2nd edition, Springer Verlag, S. 55].

Durch die nachfolgenden Beispiele soll die vorliegende Erfindung näher erläutert werden.

### Beispiele:

### Zu Schema A:

### Beispiel 1:

### Herstellung des racemischen cis-3-Hydroxycyclohexan-1-carbonsäure-isopropylesters

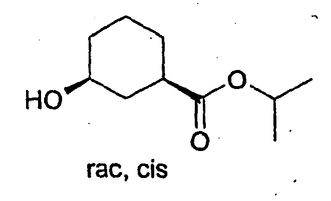

2.1 L Isopropanol wurden langsam unter Rühren mit 245 ml Acetylchlorid versetzt. Die Temperatur stieg dabei auf 45 °C, fiel aber schnell auf 35 °C ab. Dann tropfte man eine Lösung aus 350 g (2.72 mol) racemischem 6-Oxabicyclo[3.2.1] octan-7-on und 1.4 L Isopropanol langsam zu und rührte bei 20-25 °C. Nach 3 h und Stehenlassen über Nacht war die Reaktion beendet. Das Reaktionsgemisch wurde im Vakuum eingeengt, in ca. 1,3 L Methylenchlorid aufgenommen und mit 1 L halbgesättigter Natriumbicarbonatlösung gewaschen. Anschließend wurde die organische Phase mit MgSO₄ getrocknet und im Vakuum eingedampft; Ausbeute: 501 g (98.9 %); ¹H-NMR (CDCl₃): δ = 1.23 (d, 6 H), 1.20-1.45 (m, 4 H), 1.68 (d, 1 H), 1.86 (m, 2 H), 1.95 (m, 1H), 2.18 (m, 1 H), 2.34 (m, 1 H), 3.63 (m, 1 H), 5.00 (sept, 1 H).

### Beispiel 2:

### Enzymatische Racematspaltung von cis-3-Hydroxy-cyclohexan-1-carbonsäureisopropylester

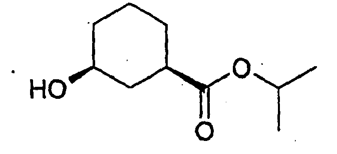

800 g des racemischen 3-Hydroxy-cyclohexan-1-carbonsäure-isopropylesters wurden mit 1.5 L Vinylacetat, 5 L Methylenchlorid und 137 g Novozym 435 bei 20-23 °C langsam gerührt. Nach etwa 4 h wurde abfiltriert und im Vakuum eingeengt. 940 g wurden erhalten und an 6 kg Kieselgel chromatographiert (n-Heptan/EE 2 : 1 - EE/n-Heptan 3:1): 1. Fraktion, 484 g, (1S,3R)-3-Acetoxy-cyclohexan-1-carbonsäureisopropylester; ¹H-NMR (CDCl₃): δ = 1.22 (d, 6 H), 1.2-1.6 (m, 4 H), 1.8-2.0 (m, 3 H), 2.03 (s, 3 H), 2.20 (m, 1 H), 2.36 (m, 1 H), 4.70 (m, 1 H), 5.00 (sept, 1 H); 80 % ee (HPLC an Chiralpak ADH 32 250 x 4.6; 1 mUmin, Heptan/ EtOH 3:1). 2. Mischfraktion. 3. Fraktion, 324 g (1R,3S)-3-Hydroxy-cyclohexan-1-carbonsäureisopropylester; ¹H-NMR (CDCl₃): δ = 1.23 (d, 6 H), 1.20-1.45 (m, 4 H), 1.68 (d, 1 H), 1.86 (m, 2 H), 1.95 (m, 1 H), 2.18 (m, 1 H), 2.34 (m, 1 H), 3.63 (m, 1 H), 5.00 (sept, 1 H); > 99 % ee (HPLC an Chiralpak ADH 32 250 x 4.6; 1 mL/min, Heptan/ EtOH 3:1).

### Beispiel 3:

### Herstellung von (1R,3S)-3-(5-Methyl-2-p-tolyl-oxazol-4-ylmethoxy)-cyclohexancarbonsäureisopropylester durch Alkylierung von (1R,3S)-3-Hydroxy-cyclohexan-1-carbonsäureisopropylester mit 4-lodmethyl-5-methyl-2-p-tolyl-oxazol

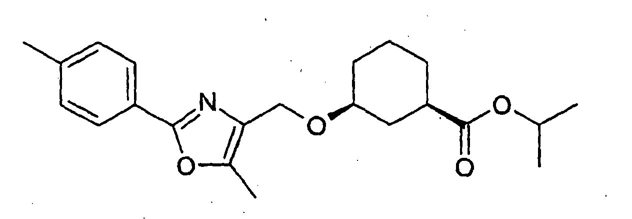

100 g (0.54 mol) (1R,3S)-3-Hydroxy-cyclohexan-1-carbonsäureisopropylester und 151 g (0.48 mol) 4-lodmethyl-5-methyl-2-p-tolyl-oxazol wurden in 1 L NMP unter N₂ vorgelegt und auf -20°C gekühlt. 20.4 g NaH wurden portionsweise innerhalb von ca. 1 h zugeben. Die Temperatur wurde dabei unter -15°C gehalten. Anschließend wurde bei -15°C gerührt. Nach 7h war die Reaktion beendet. Die Reaktionsmischung wurde in eine Mischung aus 3 L Wasser und 40 ml Eisessig gegossen. Das Produkt wurde mit MTB-Ether (2x 700 ml) extrahiert. Die organische Phase wurde im Vakuum eingeengt. 180 g Rohprodukt wurden erhalten und direkt weiter umgesetzt.

### Beispiel 4:

### Herstellung von (1R,3S)-3-(5-Methyl-2-p-tolyl-oxazol-4-ylmethoxy)-cyclohexancarbonsäure durch Verseifung des Isopropylester

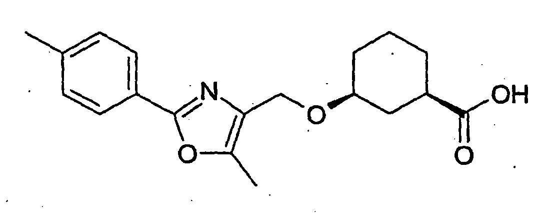

Ca. 360 g aus der zweifachen Durchführung des Beispiels 3 wurden in 1.6 L NMP gelöst, 400 mL NaOH wurden zugeben die Mischung bei RT gerührt. Nach ca. 1.5 h war die Reaktion beendet. Die Reaktionslösung wurde in 6 L Wasser gegossen, dreimal mit je 2 L MTB-Ether gewaschen und die Wasserphase mit ca. 450 ml konz. HCl auf pH 1 gestellt. Das Produkt fiel dabei aus, wurde abfiltriert, mit Wasser gewaschen. bei 50°C getrocknet; Ausbeute: 85 g; mp 144-146 °C; ¹H-NMR (CDCl₃): δ = 1.23 -1.53 (m, 4 H), 1.85 - 2.1 (m, 3 H), 2.36 - 2.45 (m, 8 H), 3.45 (m, 1 H), 4.49 (dd, 2 H), 7.23 (d, 2 H), 7.88 (d, 2 H).

### Beispiel 5:

### Herstellung von 3-Methyl-2(S)-{[(1R,3S)-3-(5-methyl-2-p-tolyl-oxazol-4-ylmethoxy)-cyclohexanecarbonyl]-amino}-buttersäure-tert-butylester durch Kupplung von (1 R,3S)-3-(5-Methyl-2-p-tolyl-oxazol-4-ylmethoxy)-cyclohexancarbonsäure mit (S)-Valin-t-butylester-hydrochlorid

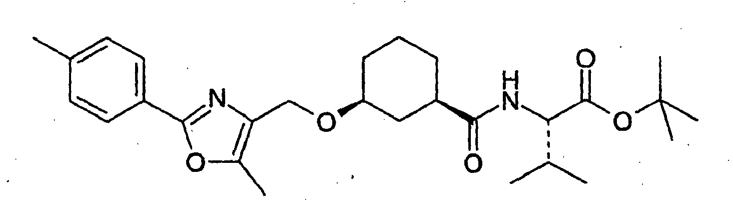

85 g (1 R,3S)-3-(5-Methyl-2-p-tolyl-oxazol-4-ylmethoxy)-cyclohexancarbonsäure wurden mit 70.4 g (S)-Valin-t-butylester-hydrochlorid und 128 mL Triethylamin in 1.39 L DMF unter Rühren vorgelegt. Man rührte ca. 10 min, kühlte dann auf 0°C (Eis-Methanol) und gab langsam portionsweise 101 g TOTU zu. Man rührte 15 min bei 0°C und anschließend bei ca. 20°C. Nach 2 h war die Reaktion beendet. Man gab alles auf 4.5 L Wasser, extrahiert 3x mit je 700 mL MTB-Ether, wusch die vereinigten organischen Phasen mit ca. 1 L Wasser um DMF-Reste zu entfernen, trocknete mit MgSO4 und engte im Vakuum ein. Der Rückstand wurde mit DIPE verrührt und abgesaugt; Ausbeute: 82 g. Die Mutterlauge wurde eingeengt und der Rückstand erneut mit DIPE verrührt; Gesamt-ausbeute: 90 g; ¹H-NMR (CDCl₃): δ = 0.87 - 0.92 (2 d, 6 H), 1.25 - 1.55 (m, 4 H), 1.46 (s, 9 H), 1.88 (m, 2 H), 2.10 - 2.35 (m, 4 H), 2.38 (s, 3 H), 2.40 (s, 3 H), 3.47 (m, 1 H), 4.46 (dd, 1 H), 4.49 (s, 2 H), 5.97 (d, 1 H), 7.22 (d, 2 H), 7.88 (d, 2 H).

### Beispiel 6:

### Herstellung von 3-Methyl-2(S)-{[(1R,3S)-3-(5-methyl-2-p-tolyl-oxazol-4-ylmethoxy)-cyclohexanecarbonyl]-amino}-buttersäure

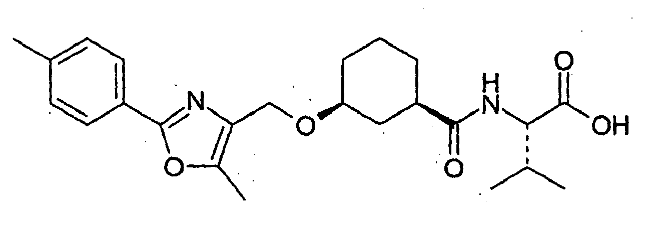

141.0 g 3-Methyl-2(S)-{[(1R,3S)-3-(5-methyl-2-p-tolyl-oxazol-4-ylmethoxy)-cyclohexanecarbonyl]-amino}-buttersäure-tert-butylester wurden in 700 mL Methylenchlorid gelöst und mit 252 mL Trifluoressigsäure versetzt. Die Reaktionsmischung wurde zum Rückfluß erhitzt. Nach ca. 10-stündiger Reaktion wurde die Reaktionslösung im Vakuum eingeengt, zweimal mit ca. 100 mL Toluol versetzt und im Vakuum eingeengt. Der resultierende Rückstand wurde in 1 L Wasser aufgenommen und mit ca. 150 mL 33 % aq. NaOH versetzt. Das Na-Salz der Carbonsäure ging in Lösung, und die Lösung wurde zweimal mit je 70 mL MTB-Ether gewaschen. Die Wasserphase wurde anschließend mit konz. HCl auf pH 1 gestellt. Das gewünschte Produkt fiel aus, wurde abgesaugt, mit Wasser gewaschen, bei 50 °C im Vakuum getrocknet und mit ca. 1 L Ethylacetat digeriert; Ausbeute: 118.8 g; mp 195-196 °C; ¹H-NMR (DMSO): δ = 0.86 (d, 6 H), 1.05-1.3 (m, 4 H), 1.64 (m, 1 H), 1.76 (m, 1 H), 2.04 (m, 4 H), 2.36 (s, 3 H), 2.38 (s, 3 H), - 3.35 (m, 1 H), 4.13 (dd, 1 H), 4.40 (s, 2 H), 7.31 (d, 2 H), 7.81 (d, 2 H), 7.84 (d, 1 H), 12.5 (s, br., 1 H).

### Beispiel 7:

### 3-Methyl-2(S)-{[(11R,3S)-3-(5-methyl-2-p-tolyl-oxazol-4-ylmethoxy)-cyclohexanecarbonyl]-amino}-pentansäure

Analog zur Reaktionssequenz Beispiel 2 - 6 wurde aus (1R,3S)-3-Hydroxycyclohexan-1-carbonsäure-isopropylester, 4-Iodmethyl-5-methyl-2-p-tolyl-oxazol und (S)-Leucin-tert-butylesterhydrochlorid das Produkt 3-Methyl-2(S)-{[(1R,3S)-3-(5-methyl-2-p-tolyl-oxazol-4-ylmethoxy)-cyclohexancarbonyl]-amino}-pentansäure erhalten; C₂₅H₃₄N₂O₅ (442.56), MS (ESI): 443 (M+H⁺).

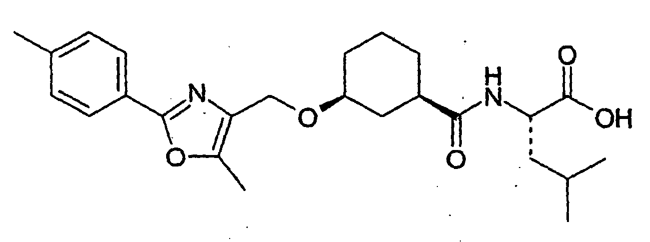

### Beispiel 8:

### 3-Methyl-2(S)-{[(1R,3S)-3-(5-methyl-2-p-tolyl-oxazol-4-ylmethoxy)-cyclohexanecarbonyl]-amino}-propionsäure

Analog zu Beispiel 7 wurde aus (1R,3S)-3-Hydroxy-cyclohexan-1-carbonsäureisopropylester, 4-Iodmethyl-5-methyl-2-p-tolyl-oxazol und (S)-Alanin-tert-butylesterhydrochlorid das Produkt 3-Methyl-2(S)-{[(1R,3S)-3-(5-methyl-2-p-tolyl-oxazol-4-ylmethoxy)-cyclohexanecarbonyl]-amino}-propionsäure erhalten; C₂₂H₂₈N₂O₅ (400.48), MS (ESI): 401 (M+H⁺).

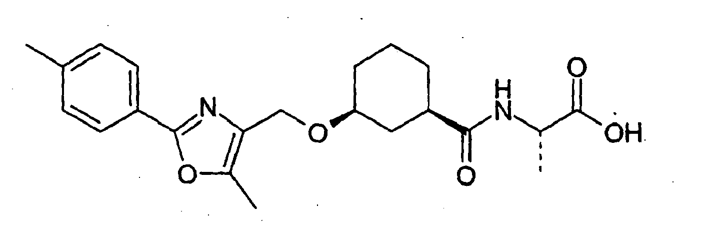

### Zu Schema B:

### Beispiel 9:

### Herstellung von (1 R,3S)-3-Hydroxy-cyclohexan-1-carbonsäure-isopropylester durch enzymatische Racematspaltung

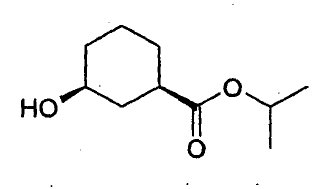

100 g des racemischen 3-Hydroxy-cyclohexan-1-carbonsäure-isopropylesters wurden mit 200 ml Vinylacetat, 800 ml Methylenchlorid und 20 g Novozym 435 bei Raumtemperatur langsam gerührt. Nach etwa 60 %igem Umsatz (GC) wurde abfiltriert und im Vakuum eingeengt. 110 g wurden erhalten und an 1 kg Kieselgel chromatographiert (n-Heptan-EE 2 : 1): 1. Fraktion, 70.9 g, (1 S,3R)- 3-Acetoxycyclohexan-1-carbonsäure-isopropylester; ¹H-NMR (CDCl₃): □ = 1.22 (d, 6 H), 1.2-1.6 (m, 4 H), 1.8-2.0 (m, 3 H), 2.03 (s, 3 H), 2.20 (m, 1 H), 2.36 (m, 1 H), 4.70 (m, 1 H), 4.99 (sept, 1 H); 62 % ee (HPLC an Chiralpak ADH 32 250 x 4.6; 1 mL/min, Heptan/EtOH 3:1). 2. Fraktion, 35.9 g (1R,3S)-3-Hydroxy-cyclohexan-1-carbonsäureisopropylester; ¹H-NMR (CDCl₃): δ = 1.23 (d, 6 H), 1.20-1.45 (m, 4 H), 1.68 (d, 1 H), 1.86 (m, 2 H), 1.95 (m, 1 H), 2.18 (m, 1 H), 2.34 (m, 1 H), 3.63 (m, 1H), 5.00 (sept, 1 H); ); > 99 % ee (HPLC an Chiralpak ADH 32 250 x 4.6; 1 mL/min, Heptan/ EtOH 3:1).

### Beispiel 10:

### Herstellung von (1 R,3S)-3-Hydroxycyclohexan-1-carbonsäure durch basische Hydrolyse von optisch reinem (1R,3S)-3-Hydroxycyclohexan-1-carbonsäureisopropylester

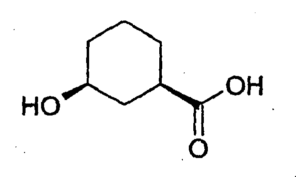

1 g (5.4 mmol) (1R,3S)-3-Hydroxy-cyclohexan-1-carbonsäure-isopropytester wurden in 6 mL 2 N NaOH bei RT über Nacht gerührt. Wenige Tropfen 11 N NaOH wurden zugegeben und erneut über Nacht gerührt. Es war kein Ester mehr nachweisbar. Man säuerte mit HCl an, engte im Vakuum ein, digerierte den Rückstand mit Isopropanol, filtrierte und engte die resultierende Lösung im Vakuum ein; Ausbeute: 0.6g ; ¹H-NMR (D₂O) in Übereinstimmung mit NMR-Daten der racemischen Säure.

### Beispiel 11:

### Herstellung von 2(S)-[((1R,3S)-3-Hydroxy-cyclohexancarbonyl)-amino]-3-methylbuttersäure tert-butylester durch Kupplung von (1R,3S)-3-Hydroxy-cyclohexan-1-carbonsäure mit (S)-Valin-tert.-butylester-hydrochlorid

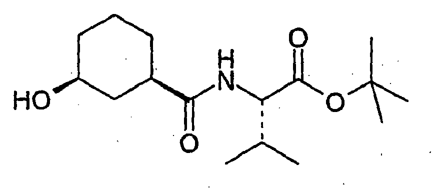

8.15 g (56.5 mmol) (1 R,3S)-3-Hydroxy-cyclohexan-1-carbonsäure, 19.6 mL Triethylamin und 11.86 g (56.5 mmol) (S)-Valin-tert.-butylester-hydrochlorid wurden in 100 mL DMF vorgelegt, auf 0 °C abgekühlt und portionsweise mit 22.2 g (67.8 mmol) TOTU ([Cyano(ethoxycarbonyl)methylenamino]-1,1,3,3-tetramethyluroniumtetrafluoroborat) versetzt. Die Reaktionslösung rührte über Nacht bei 18-22 °C. Laut LC-MS hatte das Edukt abreagiert. DMF wurde im Vakuum eingedampft, der Rückstand in Essigester aufgenommen und mit aq. NaHCO₃ gewaschen. Die org. Phase wurde getrocknet (MgSO₄) und im Vakuum eingeengt: 29.4 g eines rotbraunen Öls wurden erhalten. Zur Reinigung wurde an Kieselgel chromatographiert (n-Hep/EE 2/1 - 1/1): 11.8 g eines gelben Feststoffs wurden erhalten, der direkt weiter umgesetzt wurde; ¹H-NMR (CDCl₃): δ = 0.9 (m, 6 H), 1.25 - 1.57 (m, 4 H), 1.47 (s, 9 H), 1.78 - 1.95 (m, 3 H), 2.07 - 2.35 (m, 3 H), 3.67 (m, 1 H), 4.45 (m, 1 H), 6.0 (d, br., 1 H).

2(S)-[((1R,3S)- 3-Hydroxy-cyclohexancarbonyl)-amino]-3-methyl-buttersäure tert-butylester kann durch Alkylierung mit 4-Iodmethyl-5-methyl-2-p-tolyl-oxazol in 3-Methyl-2(S)-{[(1R,3S)-3-(5-methyl-2-p-tolyl-oxazol-4-ylmethoxy)-cyclohexanecarbonyl]-amino}-buttersäure-tert-butylester überführt werden.

### Beispiel 12:

### Herstellung von (1S,3R)-3-Hydroxycyclohexan-1-carbonsäure aus (1S,3R)-3-Acetoxycyclohexan-1-carbonsäure-isopropylester durch enzymatische Esterspaltung

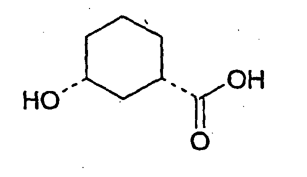

10 g (1S,3R)-3-Acetoxy-cyclohexan-1-carbonsäureisopropylester wurden 20 h bei RT in 100 mL Phosphat-Puffer, pH = 7, mit 5 g Novozym 435 gerührt. Es war kein Ester mehr nachweisbar (DC-bzw. GC-Kontrolle). Man filtrierte das immobilisierte Enzym ab, säuerte mit HCl an und engte im Vakuum ein. Der Rückstand wurde mit Isopropanol digeriert. Nach Filtration wurde die resultierende klare Lösung im Vakuum eingeengt; Ausbeute:
7.2 g; ¹H-NMR (D₂O) in Übereinstimmung mit den NMR-Daten der racemischen Säure.

### Beispiel 13:

### Herstellung von 2(R)-[((1S,3R)-3-Hydroxy-cyclohexancarbonyl)-amino]-3-methylbuttersäure-tert-butylester durch Kupplung von (1S,3R)-3-Hydroxy-cyclohexan-1-carbonsäure mit (R)-Valin-tert.-butylester-hydrochlorid

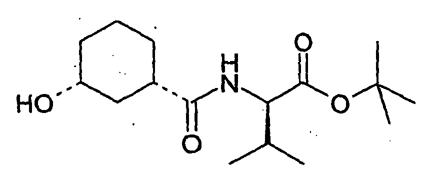

2.78 g (19.3 mmol) (1S,3R)-3-Hydroxy-cyclohexan-1-carbonsäure, 6.7 mL Triethylamin und 4.04 g (19.3 mmol) (R)-Valin-tert.-butylester-hydrochlorid wurden in 60 mL DMF vorgelegt, auf 0 °C abgekühlt und portionsweise mit 7.57 g (23.1 mmol) TOTU ([Cyano(ethoxycarbonyl)methylenamino]-1,1,3,3-tetramethyluroniumtetrafluoroborat) versetzt. Die Reaktionslösung rührte über Nacht bei 18-22 °C. DMF wurde im Vakuum eingedampft, der Rückstand in Essigester aufgenommen und mit aq. NaHCO₃ gewaschen. Die org. Phase wurde getrocknet (MgSO₄), im Vakuum und zur Reinigung an Kieselgel chromatographiert (n-Hep/EE 2/1 - 1/1): 4.96 g eines gelben Feststoffs wurden erhalten; ¹H-NMR (CDCl₃): δ = 0.9 (m, 6 H), 1.25 -1.57 (m, 4 H), 1.47 (s, 9 H), 1.78 - 1.95 (m, 3 H), 2.07 - 2.35 (m, 3 H), 3.67 (m, 1 H), 4.45 (m, 1 H), 6.0 (d, br., 1 H).

2(R)-[((1S,3R)-3-Hydroxy-cyclohexancarbonyl)-amino]-3-methyl-buttersäure-tert-butylester kann durch Alkylierung mit 4-Iodmethyl-5-methyl-2-p-tolyl-oxazol in 3-Methyl-2(R)-{[(1S,3R)-3-(5-methyl-2-p-tolyl-oxazol-4-ylmethoxy)-cyclohexanecarbonyl]-amino}-buttersäure-tert-butylester überführt werden.

### Zu Schema C:

### Beispiel 14:

### Herstellung von racemischer cis-3-Hydroxycyclohexan-1-carbonsäure durch basische Hydrolyse von rac-6-Oxabicyclo[3.2.1]octan-7-on

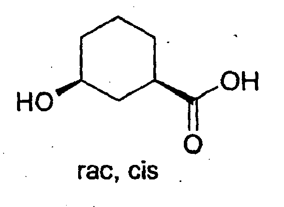

1 g (7.9 mmol) rac-6-Oxabicyclo[3.2.1] octan-7-on wurden mit 0.64 g (2 eq.) NaOH in 20 ml Wasser bei RT über Nacht gerührt. Man säuerte mit HCl oder Eisessig an, engte im Vakuum ein, digerierte den Rückstand mit Essigester oder Isopropanol und engte die resultierende organische Lösung im Vakuum ein; Ausbeute: 0.6-0.7 g ; ¹H-NMR (D₂O): δ = 0.9-1.2 (m, 4 H), 1.6-1.77 (m, 3 H), 1.95 (m, 1 H), 2.17 (m, 1 H), 3.45 (m, H).

### Beispiel 15:

### Herstellung des racemischen cis-3-Hydroxycyclohexan-1-carbonsäure-methylesters

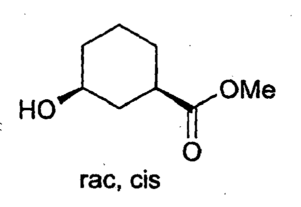

10 g (79.3 mmol) rac-6-Oxabicyclo[3.2.1] octan-7-on und 100 mL Methanol wurden langsam unter Rühren und leichter Eiskühlung mit 7 ml Acetylchlorid versetzt. Die Temperatur stieg dabei auf 45 °C, fiel aber in 10 min auf 30 °C ab. Nach 1 h war die Reaktion beendet, das Reaktionsgemisch wurde im Vakuum eingeengt; Ausbeute: 12.1 g; ¹H-NMR (CDCl₃):δ = 1.15-1.50 (m, 5 H), 1.8-2.0 (m, 3 H), 2.2 (m, 1 H), 2.37 (m, 1 H), 3.65 (m, 1 H), 3.7 (s, 3 H).

### Beispiel 16:

### Herstellung von racemischer cis-3-Hydroxycyclohexan-1-carbonsäure durch basische Hydrolyse des racemischen Methylesters

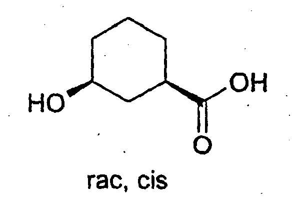

1 g (6.3 mmol) cis-3-Hydroxy-cyclohexan-1-carbonsäure-methylester wurden in 5 mL THF, 1 mL Wasser und 1 mL 11 N NaOH über Nacht bei RT gerührt. Es war kein Ester mehr nachweisbar. Man säuerte mit HCl an, engte im Vakuum ein, digerierte den Rückstand mit Essigester, filtrierte und engte die Lösung im Vakuum ein; Ausbeute:
0.7g ; ¹H-NMR (D₂O) in Übereinstimmung mit NMR-Daten des vorherigen Beispiels.

### Beispiel 17:

### Herstellung des racemischen cis-3-Hydroxycyclohexan-1-carbonsäure-benzylesters

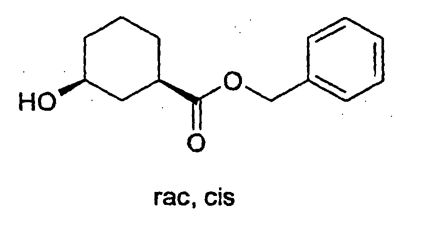

0,99 g (7.9 mmol) rac-6-Oxabicyclo[3.2.1] octan-7-on werden in 4 mL DMF mit 0.97 mL (1.2 eq.) Benzylalkohol und 1.3 g (2.2 eq.) Kaliumcarbonat bei 20-23 °C gerührt. Nach Ablauf der Reaktion wurde Wasser zugegeben und mit MTBE extrahiert. Die vereinigten organischen Phasen wurden mit ges. NaCl-Lösung gewaschen, mit MgSO₄ getrocknet und im Vakuum eingeengt. Chromatographie an Kieselgel (CH₂Cl₂ - CH₂Cl₂/Aceton 19:1 - CH₂Cl₂/MeOH 18:1) ergab 0.55 g des gewünschten Produktes; ¹H-NMR (CDCl₃) in Übereinstimmung mit ¹H-NMR aus der Umsetzung des racemischen cis-3-Hydroxycyclohexan-1-carbonsäure-Cäsiumsalzes mit Benzylbromid.

### Beispiel 18:

### Herstellung der cis-3-Hydroxycyclohexan-1-carbonsäure durch Hydrogenolyse von cis-3-Hydroxycyclohexan-1-carbonsäure-benzylester

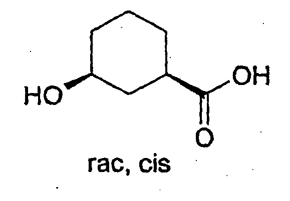

4 g (17.1 mmol) cis-3-Hydroxy-cyclohexan-1-carbonsäure-benzylester wurden in 100 mL MeOH bei RT gelöst, mit katalytischen Mengen Pd (10 % auf Kohle) versetzt und bei 5 bar hydriert. Nach Ablauf der Reaktion (TLC, LCMS) wurde der Katalysator über Celite abfiltriert und das Lösungsmittel im Vakuum eingeengt. Der trübe Rückstand wurde mit Essigsäureethylester digerierte, filtriert und die resultierende Lösung im Vakuum eingeengt; Ausbeute: 2.0 g ; ¹H-NMR (D₂O) in Übereinstimmung mit den oben genannten NMR-Daten.

### Beispiel 19:

### Herstellung von 2(S)-[((1R,3S)-3-Hydroxy-cyclohexancarbonyl)-amino]-3-methylbuttersäure tert-butylester durch Kupplung von racemischer cis-3-Hydroxy-cyclohexan-1-carbonsäure mit (S)-Valin-tert.-butylester-hydrochlorid und anschließender stereoselektiver-enzymatischer Acylierung

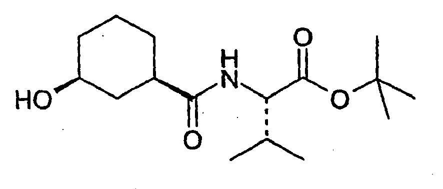

Racemische cis-3-Hydroxy-cyclohexan-1-carbonsäure und optisch reines (S)-Valin-tert.-butylester-hydrochlorid wurden in Gegenwart von Triethylamin in DMF mit TOTU ([Cyano(ethoxycarbonyl)methylenamino]-1,1,3,3-tetramethyluroniumtetrafluoroborat) gekuppelt (Reaktionsbedingungen s. Beispiel 11) und die Reaktionslösung aufgearbeitet.
10 g des resultierenden Diastereomerengemisches wurde in 300 mL Vinylacetat mit 1 g Lipase B aus Candida antarctica bei 20-23 °C gerührt. Nach etwa 53 % Umsatz wurde die stereoselektive Acylierung der Hydroxygruppe durch Abfiltrieren des Enzyms beendet. Nach Chromatographie wurden 4 g 2(S)-[((1R,3S)-3-Hydroxycyclohexancarbonyl)-amino]-3-methyl-buttersäure-tert-butylester mit 96.4 % de (HPLC an Chiralpak AD 250 x 4.6; 1 mL/min, Heptan/ EtOH/MeOH 20:1:1 + 0.1 % TFA) und 6 g des (S)-2-(1 S,3R)-Acetats mit > 80 % de erhalten.

Substanz wurde durch Alkylierung mit 4-Iodmethyl-5-methyl-2-p-tolyl-oxazol in 3-Methyl-2(S)-{[(1R,3S)-3-(5-methyl-2-p-tolyl-oxazol-4-ylmethoxy)-cyclohexanecarbonyl]-amino}-buttersäure-tert-butylester überführt.

### Zu Schema D:

### Beispiel 20:

### Synthese von (1R,3S)-3-(tert.-Butyl-diphenyl-silanyloxy)-cyclohexan-carbonsäureisoproylester

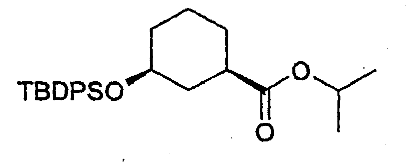

5.8 g optisch reiner (1R,3S)-3-Hydroxy-cyclohexan-1-carbonsäure-isoproylester wurden in 20 mL DMF gelöst und mit 9:74 mL TBDPSCl (tert.-Butyl-diphenylsilylchlorid), 3.2 g Imidazol und 100 mg DMAP (Dimethylaminopyridin) versetzt und 4 h bei 18-23 °C gerührt. Das Lösungsmittel wurde unter vermindertem Druck weitgehend abdestilliert, der ölige Rückstand zwischen MTBE und Wasser verteilt. Nach Trocknung der organischen Phase (MgSO₄) wurde das Lösungsmittel unter vermindertem Druck abdestilliert; Ausbeute: 14 g Rohprodukt. Chromatographie an Kieselgel (EE/n-Heptan 1:6) ergab
7.0 g (1R,3S)-3-(tert.-Butyl-diphenyl-silanyloxy)-cyclohexan-carbonsäureisopropylester;
¹H-NMR (CDCl₃): δ = 1.04 (s, 9 H), 1.19 (d, 6 H), 1.0 - 1.35 (m, 3 H), 1.48 (m, 1 H), 1.65 - 1.82 (m, 3 H), 2.06 (m, 2 H), 3.57 (m, 1 H), 4.95 (m, 1 H), 7.34 - 7. 43 (m, 6 H), 7.66 - 7. 68 (m, 4 H).

### Beispiel 21:

### Synthese von (1R,3S)-3-(tert.-Butyl-diphenyl-silanyloxy)-cyclohexan-carbonsäuremethylester

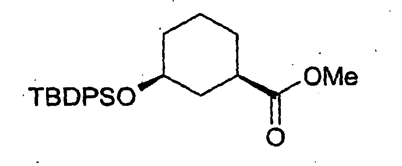

11.5 g (1 R,3S)-3-Hydroxy-cyclohexan-1-cartionsäure-methylester mit > 99 % ee wurden in 100 mL DMF gelöst und mit 21.5 g TBDPSCl (tert.-Butyl-diphenylsilylchlorid), 6.5 g Imidazol und 500 mg DMAP (Dimethylaminopyridin) versetzt und über Nacht bei
18-23 °C gerührt. Das Lösungsmittel wurde unter vermindertem Druck weitgehend abdestilliert, der ölige Rückstand in MTBE aufgenommen und mit Wasser gewaschen. Nach Trocknung der organischen Phase (MgSO₄) wurde das Lösungsmittel unter vermindertem Druck abdestilliert. Man erhielt 28 g (1R,3S)-3-(tert.-Butyl-diphenylsilanyloxy)-cyclohexan-carbonsäure-methylester als gelbliches Öl; C₂₄H₃₂O₃Si (396.61), MS (ESI): 397 (M+H⁺).

Substanz kann direkt in die basische Esterverseifung, und die anschließende Kupplung mit Aminosäurederivaten wie z. B. Alanin-, Leucin- und Valin-tert.-butylester bzw. das entsprechende Hydrochlorid eingesetzt werden.

### Beispiel 22:

### Zweistufige Synthese von 2(S)-{[(1R,3S)-3-Hydroxy-cyclohexanecarbonyl]-amino}-(3S)-methyl-buttersäure-tert-butylester aus (1R,3S)-3-(tert-Butyl-diphenyl-silanyloxy)-cyclohexan-1-carbonsäure

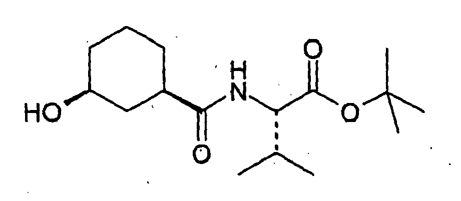

0.35 g optisch reine (1 R,3S)-3-(tert.-Butyl-diphenyl-silanyloxy)-cyclohexancarbonsäure (hergestellt durch Hydrolyse z. B. des entsprechenden Isopropylesters mit NaOH in Wasser/Isopropanol) wurden mit 0.38 g (S)-Valin-t-butylester-Hydrochlorid und 0.45 ml Triethylamin in 4 ml DMF unter Rühren vorgelegt. Bei 0-5°C gab man 0.36 g TOTU ([Cyano(ethoxycarbonyl)methylenamino]-1,1,3,3-tetramethyluroniumtetrafluoroborat) in kleinen Portionen zu, ließ ca. 5 Minuten gekühlt nachrühren. Danach rührte man weiter bei RT. Die Reaktion war nach 16 h beendet. Man gab alles in ca. 60 ml Wasser, schüttelte zweimal mit je 50 ml Essigester aus, trocknete die organische Phase mit MgSO₄ und engte im Vakuum ein. Der Rückstand (1.4 g) wurde an Kieselgel (n-Heptan-EE 1:1) gereinigt; Ausbeute: 394 mg eines weißen Feststoffs.

Zur Abspaltung der TBDPS-Schutzgruppe wurde 2(S)-{[(1R,3S)-3-(tert-Butyl-diphenylsilanyloxy)-cyclohexanecarbonyl]-amino}-3-methyl-buttersäure-tert-butylester in THF mit Tetrabutylammoniumfluorid umgesetzt. Einengen der Reaktionsmischung und Reinigung an Kieselgel (n-Heptan/EE 3:1) ergab 197 mg der gewünschte Verbindung; C₁₆H₂₉NO₄ (299.41), MS (ESI): 300 (M+H⁺); HPLC (Chiralpak AD 250 x 4.6; 1 mL/min, Heptan/ EtOH/MeOH 20:1:1 + 0.1 % TFA): Rt = 4.9 min.

### Zu Schema A, B und D

### Beispiel 23:

### Herstellung von (1 S,3R)-3-Hydroxycyclohexan-1-carbonsäure-methylester aus (1S,3R)-3-Acetoxy-cyclohexan-1-carbonsäure-isopropylester- über (1S,5R)-6-Oxabicyclo[3.2.1]octan-7-on

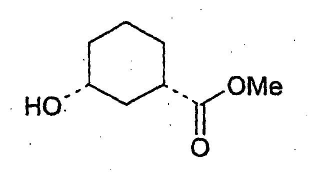

2.74 g (1S,3R)-3-Acetoxy-cyclohexan-1-carbonsäure-isopropylester, aus der stereoselektiven enzymatischen Esterbildung von racemischem cis-3-Hydroxycyclohexan-1-carbonsäure-isopropylester in Vinylacetat/Methylenchlorid mit Novozym 435, wurden durch Rühren mit 0.3 g K₂CO₃ in 30 mL Methanol bei 20-23 °C in in nahezu quantitativer Ausbeute in (1S,5R)-6-Oxabicyclo[3.2.1]octan-7-on überführt. Nach Filtration und Einengen des Lösungsmittels im Vakuum wurde (1 R,5S)-6-Oxabicyclo[3.2.1] octan-7-on

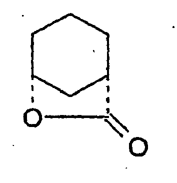

in ca. 30 mL Methanol aufgenommen und unter Rühren und leichter Eiskühlung mit 1-2 ml Acetylchlorid versetzt. Nach Ablauf der Reaktion wurde das Reaktionsgemisch im Vakuum eingeengt; Ausbeute: 1.5 g; ¹H-NMR (CDCl₃):δ = 1.15-1.50 (m, 5 H), 1.8-2.0 (m, 3 H), 2.2 (m, 1 H), 2.37 (m, 1 H), 3.65 (m, 1 H), 3.7 (s, 3 H).

(1S,3R)-3-Hydroxycyclohexan-1-carbonsäure-methylester wurde direkt weiter umgesetzt.

Zu Schema A, B, C, D, E - Zweistufige Überführung der Verbindung der Formel (II) in eine racemische Verbindung der Formel (IV)

### Beispiel 24:

### Herstellung des racemischen cis-3-Hydroxycyclohexan-1-carbonsäure-Cäsiumsalzes

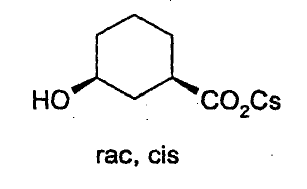

10 g (79.3 mmol) rac-6-Oxa-bicycio[3.2.1]octan-7-on werden mit 13,5 g (80.3 mmol) Cäsiumhydroxid-Monohydrat und 50 ml Wasser bei RT gerührt. Nach 2 h war die Umsetzung beendet. Man engte im Vakuum ein, gab zweimal jeweils 50 mL DMF zu und engte im Vakuum ein; Ausbeute: 19.6 g (89.5 %); ¹H-NMR (D₂O): □ = 1.1-1.37 (m, 4 H), 1.75-2.25 (m, 5 H), 3.63 (m, 1 H).

### Beispiel 25:

### Herstellung des racemischen cis-3-Hydroxycyclohexan-1-carbonsäure-benzylesters

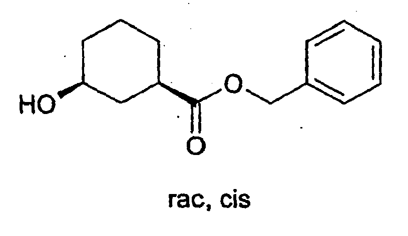

2 g (7.24 mmol) racemisches cis-3-Hydroxycyclohexan-1-carbonsäure-Cäsiumsalz wurden mit 1,1 g (0,81 ml, 6.82 mmol) Benzylbromid und 10 ml DMF bei RT gerührt. Nach 4-stündigem Rühren und Stehenlassen über Nacht war die Benzylierung beendet. Das Reaktionsgemisch wurde auf etwa 100 mL Wasser gegeben und zweimal mit jeweils ca. 50 mL MTBE extrahiert. Die vereinigten organischen Phasen wurden einmal mit Wasser gewaschen und dann mit MgSO₄ getrocknet und im Vakuum eingeengt; Rohausbeute: 1.3 g (76.6 %). Das Rohprodukt wurde entweder direkt weiter umgesetzt oder chromatographiert; ¹H-NMR (CDCl₃): δ = 1.17-1.50 (m, 4 H), 1.62 (d, 1 H), 1.80-2.0 (m, 3 H), 2.22 (m, 1 H), 2.43 (m, 1H), 3.62(m, 1 H), 5.12 (s, 2 H), 7.28-7.40 (m, 5 H).

Weitere Beispiele für die enzymatisch Racematspaltung durch stereoselektive Esterbildung

### Beispiel 26:

### Stereoselektive Acylierung von cis-3-Hydroxy-cyclohexan-1-carbonsäure-methylester

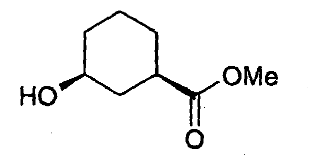

500 mg des racemischen 3-Hydroxy-cyclohexan-1-carbonsäure-methylesters wurden mit 1 ml Vinylacetat, 4 ml Methylenchlorid und 25 m g Novozym 435 bei Raumtemperatur langsam gerührt. Nach etwa 54 %igem Umsatz (GC) wurde die Reaktion durch Abfiltrieren des Enzyms beendet. Die Bestimmung der optischen Reinheit von (1 R,3S)-3-Hydroxy-cyclohexan-1-carbonsäure-methylester ergab > 99 % ee.

### Beispiel 27:

### Synthese von (1R,3S)-3-Hydroxy-cyclohexan-1-carbonsäure-benzyester durch stereoselektive Acylierung von racemischem cis-3-Hydroxy-cyclohexan-1-carbonsäure-benzylester

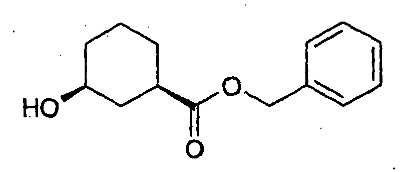

108 mg (0.5 mmol) des racemischen 3-Hydroxy-cyclohexan-1-carbonsäurebenzylesters wurden 2 h in 5 ml Vinylacetat und 4 ml Methylenchlorid mit 54 mg Novozym 435 bei Raumtemperatur langsam gerührt. Nach Filtration und Einengen im Vakuum wurde an Kieselgel chromatographiert (n-Heptan/EE 2 : 1); Ausbeute: 56 mg (1R,3S)-3-Hydroxy-cyclohexan-1-carbonsäure-benzyester; ¹H-NMR (CDCl₃): δ = 1.15-1.5 (m, 4 H), 1.63
(d, 1 H), 1.85 (m, 1 H), 1.95 (m, 2 H), 2.23 (m, 1 H), 2.41 (m, 1 H), 3.62 (m, 1 H), 5.11 (s, 2 H), 7.35 (m, 5 H); ee > 99 % (HPLC an Chiracel OJ 250 x 4.6; 1 mL/min, Heptan/ EtOH/MeOH 70:1:1).

### Beispiel 28:

### Synthese von (1R,3S)-3-Hydroxy-cyclohexan-1-carbonsäure-benzyester durch Racematspaltung von racemischem cis-3-Hydroxy-cyclohexan-1-carbonsäurebenzylester

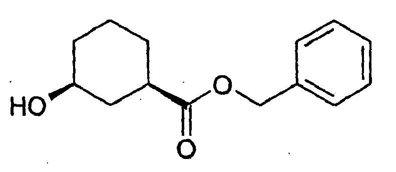

29.5 g cis-(1RS,3SR)-3-Hydroxy-cyclohexancarbonäure-benzylester wurden in ca. 200 mL Vinylacetat gelöst, mit 15 g Novozym 435 versetzt und bei 20-23 °C gerührt. Nach 75 Minuten wurde das Enzym abfiltriert und die Lösung im Vakuum eingeengt. Nach Chromatographie an Kieselgel (n-Heptan/Essigester 2:1 erhielt man 12 g (1 R,3S)-3-Hydroxy-cyclohexancarbonäure-benzylester; > 99 % ee (HPLC an Chiracel OJ 250 x 4.6; 1 mUmin, Heptan/ EtOH/CH₃OH 70:1:1); ¹H-NMR (DMSO), δ = 0.98-1.30 (m, 4 H), 1.66-1.82 (m, 4 H), 2.04 (m, 1 H); 2.39 (m, 1 H), 3.39 (m, 1 H), ), 4.63 (dd, 2 H), 5.08 (m, 1 H), 7.30-7.40 (m, 5 H).

Darüber hinaus wurden 17 g der (1S,3R)-Acetylverbindung erhalten; 94 % ee ((HPLC an Chiracel OJ 250 x 4.6; 1 mL/min, Heptan/ EtOH/CH₃OH 70:1:1, nach Abspaltung der Acetylgruppe).

### Beispiel 29

### Racematspaltung von cis-3-Hydroxy-cyclohexan-1-carbonsäure-isopropylester

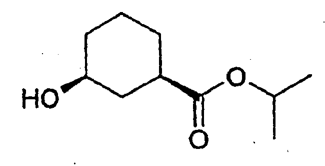

200 mg (1.07 mmol) des racemischen cis-3-Hydroxy-cyclohexan-1-carbonsäureisopropylesters wurden in 10 mL Aceton gelöst, mit 584 mg (5.78 mmol) Bernsteinsäureanhydrid und 40 mg Novozym 435 versetzt und bei 5 °C gerührt. Nach 40-45 % Umsatz wurde die Reaktion durch Abfiltrieren des Enzyms beendet. Aus einer eingeengten Probe wurde die optische Reinheit sowohl des nicht umgesetzten Substrates als auch des gebildeten Acylierungsproduktes bestimmt. Die optische Reinheit von (1 R,3S)-3-Hydroxy-cyclohexan-1-carbonsäure-isopropylester betrug 72 % ee (HPLC an Chiralpak AD-H 250 x 4.6; 1 mL/min, Heptan/MeOH/EtOH/TFA 500:100:100:0.7), die optische Reinheit des (1R,3S)-Bemsteinsäuremono-(cis-3-isopropoxycarbonyl-cyclohexyl)esters betrug > 97 % ee (HPLC an Chiralpak AD-H 250 x 4.6; 1 mL/min, Heptan/MeOH/EtOH/TFA 500:100:100:0.7).

### Beispiel 30

### Racematspaltung von cis-3-Hydroxy-cyclohexan-1-carbonsäure-isopropylester

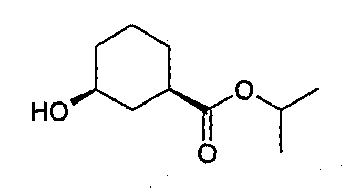

200 mg (1.07 mmol) des racemischen cis-3-Hydroxy-cyclohexan-1-carbonsäureisopropylesters wurden in 10 mL DIPE gelöst, mit 584 mg (5.78 mmol) Bernsteinsäureanhydrid und 40 mg Novozym 435 versetzt und bei 35 °C gerührt. Nach etwa 40 % Umsatz wurde die Reaktion durch·Abfiltrieren des Enzyms beendet. Aus einer eingeengten Probe wurde die optische Reinheit sowohl des nicht umgesetzten Substrates als auch des gebildeten Acylierungsproduktes bestimmt. Die optische Reinheit von (1R,3S)-3-Hydroxy-cyclohexan-1-carbonsäure-isopropylester betrug 61 % ee (HPLC an Chiralpak AD-H 250 x 4.6; 1 mL/min, Heptan/MeOH/EtOH/TFA 500:100:100:0.7), die optische Reinheit des (1R,3S)-Bernsteinsäuremono-(cis-3-isopropoxycarbonyl-cyclohexyl)esters betrug 94 % ee (HPLC an Chiralpak AD-H 250 x 4.6; 1 mL/min, Heptan/MeOH/EtOH/TFA 500:100:100:0.7).

### Beispiel 31

### Racematspaltung von cis-3-Hydroxy-cyclohexan-1-carbonsäure-isopropylester

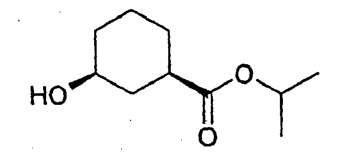

200 mg (1.07 mmol) des racemischen cis-3-Hydroxy-cyclohexan-1-carbonsäureisopropylesters wurden in 10 mL Aceton gelöst, mit 584 mg (5.78 mmol) Bernsteinsäureanhydrid und 160 mg Novozym 435 versetzt und bei 35 °C gerührt. Nach 45-49 % Umsatz wurde die Reaktion durch Abfiltrieren des Enzyms beendet. Aus einer eingeengten Probe wurde die optische Reinheit sowohl des nicht umgesetzten Substrates als auch des gebildeten Acylierungsproduktes bestimmt. Die optische Reinheit von (1R,3S)-3-Hydroxy-cyclohexan-1-carbonsäure-isopropylester betrug 84 % ee (HPLC an Chiralpak AD-H 250 x 4.6; 1 mL/min, Heptan/MeOH/EtOH/TFA 500:100:100:0.7), die optische Reinheit des (1R,3S)-Bemsteinsäuremono-(cis-3-isopropoxycarbonyl-cyclohexyl)esters betrug 96 % ee (HPLC an Chiralpak AD-H 250 x 4.6; 1 mL/min, Heptan/MeOH/EtOH/TFA 500:100:100:0.7).

### Beispiel 32

### Racematspaltung von cis-3-Hydroxy-cyclohexan-1-carbonsäure-isopropylester

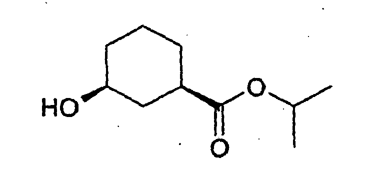

200 mg (1.07 mmol) des racemischen cis-3-Hydroxy-cyclohexan-1-carbonsäureisopropylesters wurden in 10 mL MTB-Ether gelöst, mit 119 mg (1.18 mmol) Bernsteinsäureanhydrid und 160 mg Novozym 435 versetzt und bei 35 °C gerührt. Nach 33-37 % Umsatz wurde die Reaktion durch Abfiltrieren des Enzyms beendet. Aus einer eingeengten Probe wurde die optische Reinheit sowohl des nicht umgesetzten Substrates als auch des gebildeten Acylierungsproduktes bestimmt. Die optische Reinheit von (1 R,3S)-3-Hydroxy-cyclohexan-1-carbonsäure-isopropylester betrug 46 % ee (HPLC an Chiralpak AD-H 250 x 4.6; 1 mL/min, Heptan/MeOH/EtOH/TFA 500:100:100:0.7), die optische Reinheit des (1R,3S)-Bernsteinsäuremono-(cis-3-isopropoxycarbonyl-cyclohexyl)esters betrug 93 % ee (HPLC an Chiralpak AD-H 250 x 4.6; 1 mL/min, Heptan/MeOH/EtOH/TFA 500:100:100:0.7).

### Beispiel 33

### Racematspaltung von cis-3-Hydroxy-cyclohexan-1-carbonsäure-isopropylester

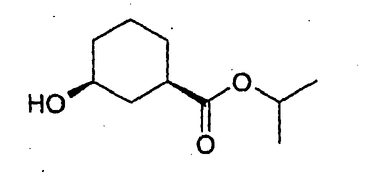

200 mg (1.07 mmol) des racemischen cis-3-Hydroxy-cyclohexan-1-carbonsäureisopropylesters wurden in 10 mL THF gelöst, mit Novozym 435 L-2 versetzt und bei 35 °C gerührt. Nach ca. 40 % Umsatz wurde die Reaktion durch Abfiltrieren des Enzyms beendet. Aus einer eingeengten Probe wurde die optische Reinheit sowohl des nicht umgesetzten Substrates als auch des gebildeten Acylierungsproduktes bestimmt. Die optische Reinheit von (1R,3S)-3-Hydroxy-cyclohexan-1-carbonsäureisopropylester betrug 64 % ee (HPLC an Chiralpak AD-H 250 x 4.6; 1 mL/min, Heptan/MeOH/EtOH/TFA 500:100:100:0.7), die optische Reinheit des (1R,3S)-Bemsteinsäuremono-(cis-3-isopropoxycarbonyl-cyclohexyl)esters betrug 97 % ee (HPLC an Chiralpak AD-H 250 x 4.6; 1 mL/min, Heptan/MeOH/EtOH/TFA 500:100:100:0.7).

### Beispiel 34

### Racematspaltung von cis-3-Hydroxy-cyclohexan-1-carbonsäure-isopropylester

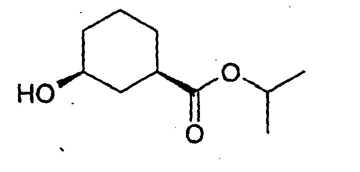

200 mg (1.07 mmol) des racemischen cis-3-Hydroxy-cyclohexan-1-carbonsäureisopropylesters wurden in 10 mL DIPE gelöst, mit 584 mg (5.78 mmol) Bernsteinsäureanhydrid und 40 mg Novozym 435 versetzt und bei 35 °C gerührt. Nach 40-45 % Umsatz wurde die Reaktion durch Abfiltrieren des Enzyms beendet.

Aus einer eingeengten Probe wurde die optische Reinheit sowohl des nicht umgesetzten Substrates als auch des gebildeten Acylierungsproduktes bestimmt. Die optische Reinheit von (1R,3S)-3-Hydroxy-cyclohexan-1-carbonsäure-isopropylester betrug 70 % ee (HPLC an Chiralpak AD-H 250 x 4.6; 1 mL/min, Heptan/MeOH/EtOH/TFA 500:100:100:0.7), die optische Reinheit des (1R,3S)-. Bernsteinsäuremono-(cis-3-isopropoxycarbonyl-cyclohexyl)esters betrug 92 % ee (HPLC an Chiralpak AD-H 250 x 4.6; 1 mL/min, Heptan/MeHO/EtOH/TFA 500:100:100:0.7).

### Beispiel 35

### Racematspaltung von cis-3-Hydroxy-cyclohexan-1-carbonsäure-isopropylester

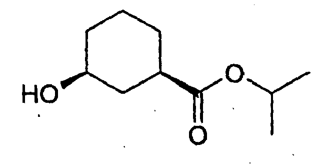

200 mg (1.07 mmol) des racemischen cis-3-Hydroxy-cyclohexan-1-carbonsäureisopropylesters wurden in 10 mL n-Heptan gelöst, mit 119 mg (1.18 mmol) Bernsteinsäureanhydrid und 160 mg Novozym 435 versetzt und bei 5 °C gerührt. Bei etwa 30 % Umsatz wurde die Reaktion durch Abfiltrieren des Enzyms beendet. Aus einer eingeengten Probe wurde die optische Reinheit sowohl des nicht umgesetzten Substrates als auch des gebildeten Acylierungsproduktes bestimmt. Die optische Reinheit von (1R,3S)-3-Hydroxy-cyclohexan-1-carbonsäure-isopropylester betrug 43 % ee (HPLC an Chiralpak AD-H 250 x 4.6; 1 mL/min, Heptan/MeOH/EtOH/TFA 500:100:100:0.7), die optische Reinheit des (1R,3S)-Bemsteinsäuremono-(cis-3-isopropoxycarbonyl-cyclohexyl)esters betrug
96 % ee (HPLC an Chiralpak AD-H 250 x 4.6; 1 mL/min, Heptan/MeOH/EtOH/TFA 500:100:100:0.7).

### Beispiel 36

### Racematspaltung von cis-3-Hydroxy-cyclohexan-1-carbonsäure-isopropylester

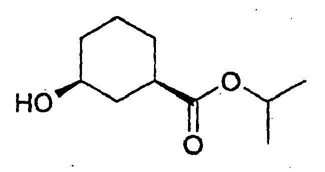

200 mg (1.07 mmol) des racemischen cis-3-Hydroxy-cyclohexan-1-carbonsäureisopropylesters wurden in 10 mL Toluol gelöst, mit 584 mg (5.78 mmol) Bernsteinsäureanhydrid und 160 mg Novozym 435 versetzt und bei 5 °C gerührt. Bei etwa 46-49 % Umsatz wurde die Reaktion durch Abfiltrieren des Enzyms beendet. Aus einer eingeengten Probe wurde die optische Reinheit sowohl des nicht umgesetzten Substrates als auch des gebildeten Acylierungsproduktes bestimmt. Die optische Reinheit von (1 R,3S)-3-Hydroxy-cyclohexan-1-carbonsäure-isopropylester betrug > 76 % ee (HPLC an Chiralpak AD-H 250 x 4.6; 1 mL/min, Heptan/MeOH/EtOH/TFA 500:100:100:0.7), die optische Reinheit des (1R,3S)-Bernsteinsäuremono-(cis-3-isopropoxycarbonyl-cyclohexyl)esters betrug
91 % ee (HPLC an Chiralpak AD-H 250 x 4.6; 1 mL/min, Heptan/MeOH/EtOH/TFA 500:100:100:0.7).

### Beispiel 37

### Herstellung von (1R,3S)-3-Hydroxy-cyclohexan-1-carbonsäure-isopropylester und (1 R,3S)-Bemsteinsäuremono-(3-isopropoxycarbonyl-cyclohexyl)ester durch stereoselektive enzymatische Acylierung von cis-3-Hydroxy-cyclohexan-1-carbonsäure-isopropylester mit Bernsteinsäureanhydrid.

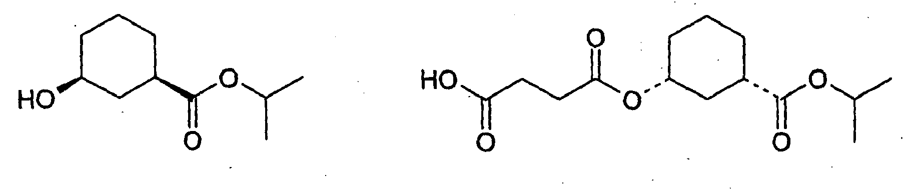

2.0 mg (10.7 mmol) des racemischen cis-3-Hydroxy-cyclohexan-1-carbonsäureisopropylesters wurden in 100 mL MTB-Ether gelöst, mit 5.84 g (57.8 mmol) Bernsteinsäureanhydrid und 1.6 g Novozym 435 versetzt und bei 35 °C gerührt. Bei ca. 50 % Umsatz wurde die Reaktion durch Abfiltrieren des Enzyms beendet. Das Reaktionsgemisch wurde in Aceton gelöst, i. V. eingeengt und dann zwischen Diisopropylether/n-Heptan 4:1 und 1 M Na₂CO₃ (aq.) verteilt. Einengen der organischen Phase ergab 890 mg (1R,3S)-3-Hydroxy-cyclohexan-1-carbonsäureisopropylester; ¹H-NMR (CDCl₃): δ = 1.23 (d, 6 H), 1.20-1.45 (m, 4 H), 1.68 (d, 1 H), 1.86 (m, 2 H), 1.95 (m, 1 H), 2.18 (m, 1 H), 2.34 (m, 1 H), 3.63 (m, 1 H), 5.00 (sept, 1 H); optische Reinheit: 90 % ee (HPLC an Chiralpak AD-H 250 x 4.6; 1 mUmin, Heptan/MeOH/EtOH/TFA 500:100:100:0.7).
Die wässrige Phase wurde mit 2 N HCl auf pH 6-7 eingestellt und zweimal mit Essigsäureethylester extrahiert. Einengen nach Trocknung (Na₂SO₄) ergab 1.39 g (1R,35)-Bemsteinsäuremono-(3-isopropoxycarbonyl-cyclohexyl)ester;
¹H-NMR (CDCl₃): δ = 1.22 (d, 6 H), 1.24-1.41 (m, 3H), 1.47 (q, 1 H), 1.83-2.01 (m, 3 H), 2.17-2.24 (m, 1 H), 2.35 (tt, 1 H), 2.58-2.64 (m, 2 H), 2.64-2.71 (m, 2 H), 4.69-4.79 (m, 1 H), 4.99 (sept, 1 H); optische Reinheit: 88 % ee (HPLC an Chiralpak AD-H 250 x 4.6; 1 mL/min, Heptan/MeOH/EtOH/TFA 500:100:100:0.7).

Isolierten Produkte bzw. Rohproduktgemische wurden durch ¹H-NMR- und/oder Massen-Spektren bzw. per GC oder HPLC identifiziert.
Die optische Reinheit der Ester und Alkohole wurde durch HPLC, z. B an Chiralpak AD 250 X 4.6 (Daicel) bzw. Chiracel OD 250 x 4,6 bestimmt.

## Patentansprüche

1. Verfahren zur Herstellung chiraler, nicht racemischer Verbindungen der Formeln (Ia) und (Ib) 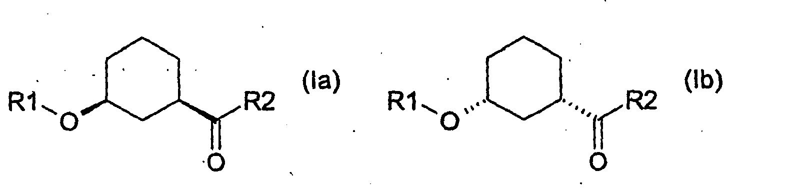 mit:
R¹ 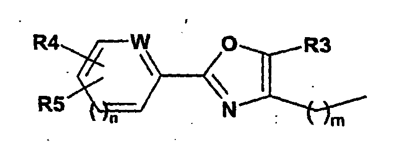 worin bedeuten:
R³ H, (C1-C6)-Alkyl, (C3-C8)-Cycloalkyl, (C1-C3)-Alkyl-(C3-C8)-Cycloalkyl, Phenyl, (C1-C3)-Alkyl-Phenyl, (C5-C6)-Heteroaryl, (C1-C3)-Alkyl-(C5-C6)-Heteroaryl oder (C1-C3)-Alkyl, das durch F ganz oder teilweise substituiert ist;
R⁴, R⁵ unabhängig voneinander H, F, Cl, Br, CF₃, OCF₃, (C1-C6)-Alkyl, O-(C1-C6)-Alkyl, SCF₃, SF₅, OCF₂-CHF₂, (C6-C10)-Aryl, (C6-C10)-Aryloxy, OH, NO₂; oder
R⁴ und R⁵ zusammen genommen mit den sie tragenden C-Atomen einen kondensierten, teilweise oder nicht gesättigten bicyclischen (C6-C10)-Aryl- oder (C5-C11)-Heteroaryl-Ring bilden;
W CH, N, falls n = 1;
W O, S, NR⁶, falls n = 0;
m 1-6;
R⁶ H, (C1-C6)-Alkyl-Phenyl, (C1-C6)-Alkyl;
oder
R¹ eine OH-Schutzgruppe (SG), wie z.B. Benzyloxymethyl, Benzyl, para-Methoxybenzyl, tert.-Butyldimethylsilyl (TBDMS), tert.-Butyldiphenylsilyl (TBDPS), Tetrahydropyranyl (THP), 1-Ethoxyethyl (EE), 1-Methyl-1-methoxyethyl oder 1-Methyl-1-benzyloxyethyl;
und
R² 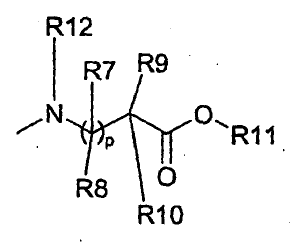 worin bedeuten:
p 0-2;
R⁷ H, (C1-C6)-Alkyl;
R⁸ H, (C1-C6)-Alkyl;
R⁹ H, F, (C1-C6)-Alkyl;
R¹⁰ H, F, (C1-C6)-Alkyl, O-(C1-C6)-Alkyl, (C2-C6)-Alkenyl, (C2-C6)Alkinyl, (C3-C8)Cycloalkyl, Phenyl, wobei Alkyl, Alkenyl, Alkinyl und Cycloalkyl gegebenenfalls substituiert sein kann durch einen oder mehrere Reste aus der Reihe Hydroxy; Phenyl, (C5-C11)-Heteroaryl, O-(C1-C6)-Alkyl und NR¹³R¹⁴, und Phenyl gegebenfalls substituiert sein kann durch einen oder mehrere Reste aus der Reihe Hydroxy, O-(C1-C6)-Alkyl, F und CF₃, mit der Maßgabe, dass R¹⁰ ungleich NR¹³R¹⁴ oder O-(C1-C6)-Alkyl, falls R⁹ = F ist;
R⁹ und R¹⁰ zusammen mit dem sie tragenden C-Atom (C3-C8)-Cycloalkyl;
R¹⁰ und R¹² zusammen Pyrrolidin oder Piperidin, falls p= 0 ;
R¹¹ H, (C1-C8)-Alkyl, Benzyl, (C1-C4)-Alkyl-(C6-C10)-Aryl, (C1-C4)-Alkyl-O-(C1-C4)-Alkyl, Phenyl-(C1-C4)-Alkyl, wobei Alkyl, Benzyl, Phenyl, Aryl, gegebenenfalls ein- oder mehrfach substituiert sein können durch O-(C1-C6)-Alkyl, OCH₂CH₂-OMe, F, Cl, Br, I, Si(CH₃)₃, OSi(CH₃)₃, Si(iPr)₃, OSi(iPr)₃, OCH₂CH₂-SiMe₃, OCH₂-Si(iPr)₃, O-CH₂-C₆H₅, SO₂C₆H₄-p-Me, SMe, CN, NO₂, CH₂COC₆H₅;
R¹² H, (C1-C6)-Alkyl, (C2-C6)-Alkenyl, (C2-C6)-Alkinyl, Benzyl, CO-(C1-C6)-Alkyl, CO-Phenyl, C(O)-O-(C1-C6)-Alkyl, Allyloxycarbonyl (ALOC), Benzyloxycarbonyl (Cbz, Z), 9-Fluorenytmethyloxycarbonyl (FMOC), (C1-C4)Alkyl-(C6-C10)-Aryl, (C1-C4)-Alkyl-(C5-C11)-Heteroaryl, (C1-C4)-Alkyl-O-(C1-C4)-Alkyl, Phenyl-(C1-C4)-Alkyl, (C5-C6)-Heteroaryl-(C1-C4)-Alkyl; SO2-(C1-C6)-Alkyl, SO2-(C1-C6)-Alkyl-SO₂(C1-C6)-alkyl, SO₂-Phenyl, wobei Phenyl gegebenenfalls substituiert sein kann durch (C1-C6)-Alkyl, O-(C1-C6)-Alkyl, F, Cl;
R¹³ (C1-C6)-Alkyl;
R¹⁴ (C1-C6)-Alkyl-Phenyl, (C1-C6)-Alkyl,
**dadurch gekennzeichnet, dass** man
**A)**
a) Lactonöffnung(LO)
racemisches 6-Oxabicyclo[3.2.1]octan-7-on der Formel (II) 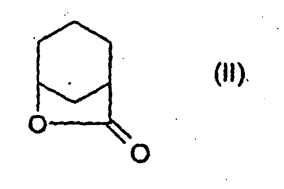 mit einer Verbindung der Formel (III)
HO-R¹⁵ (III)
mit
R¹⁵ H, (C1-C8)-Alkyl, (C3-C8)-Cycloalkyl (C2-C8)-Alkenyl, (C2-C8)-Alkinyl, Benzyl, (C1-C4)-Alkyl-(C6-C10)-Aryl, (C1-C4)-Alkyl-(C5-C11)-Heteroaryl, (C1-C4)-Alkyl-O-(C1-C4)-Alkyl, Phenyl-(C1-C4)-Alkyl, (C5-C6)-Heteroaryl-(C1-C4)-Alkyl, wobei Alkyl, Benzyl, Phenyl, Aryl, Heteroaryl gegebenenfalls ein- oder mehrfach substituiert sein können durch Phenyl, O-(C1-C6)-Alkyl, OCH₂CH₂-OMe, OTs, F, Cl, Br, I, Si(CH₃)₃, OSi(CH₃)₃, Si(iPr)₃, OSi(iPr)₃, OCH₂CH₂-SiMe₃, OCHrSi(iPr)₃, OTHP, O-CH₂-C₆H₅, SO₂C₆H₄-p-Me, SMe, CN, NO₂, COOH, CONH₂, CH₂COC₆H₅, CO-Benzoxy, CO-O(C1-C6)-Alkyl, NHTs, NHAc, NHBoc, NHAloc, NHBenzyl;
in Gegenwart von geeigneten Basen oder Säuren in einem geeigneten Lösungsmittel umsetzt oder mit Säurehalogeniden in Alkoholen, wobei im Falle der wäßrigen Reaktionen die Bedingungen der Aufarbeitung darüber entscheiden, ob man das Salz oder aber die freie Säure erhält,
z. B. zu einer racemischen cis-konfigurierten Verbindung der Formel (IV), 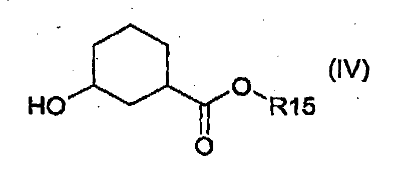 worin R¹⁵ wie oben definiert ist oder zu einer Verbindung der Formel (IV), die je nach Aufarbeitung in ionischer Form, z.B. als Gs⁺-, Li⁺-, K⁺-, NH₄⁺-, Ca²⁺-, Ba²⁺-, Mg²⁺- Salz vorliegen kann und worin R¹⁵ auch Cs, Li, K, NH₄, Ca, Ba, Mg bedeutet, und gegebenenfalls das so erhaltene Produkt zu einem anderen Produkt der Formel (IV) weiter umsetzt;
b) Enzymatische Esterbildung (EB) + Trennung (T)
die erhaltenen Verbindungen der Formyl (IV) einer stereoselektiven enzymatischen Esterbildung (EB) unterwirft, wobei die Hydroxyverbindungen in einem organischen Lösungsmittel mit einem Acyldonor und dem Enzym aus der Gruppe der Hydrolasen versetzt und die resultierende Mischung bei -20 bis 80°C gerührt wird und nach Ablauf der Reaktion das eine Stereoisomere als Ester der Formel (Vb) 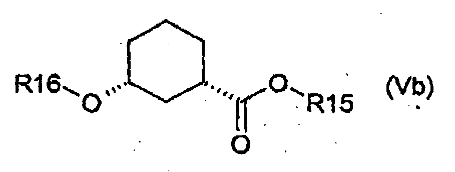 worin
R¹⁶ C(=O)-(C₁-C₁₆)-Alkyl, C(=O)-(C₂-C₁₆)-Alkenyl, C(=O)-(C₃-C₁₆)-Alkinyl, C(=O)-(C₃-C₁₆)-Cycloalkyl, wobei ein oder mehrere Kohlenstoffatome durch Sauerstoffatome ersetzt sein und substituiert sein können mit 1-3 Substituenten aus der Gruppe F, Cl, Br, CF₃, CN, NO₂, Hydroxy, Methoxy, Ethoxy, Phenyl, CO-O(C₁-C₄)-Alkyl und CO-O(C₂-C₄)-Alkenyl, wobei Phenyl, CO-O(C₁-C₄)-Alkyl und CO-O(C₂-C₄)-Alkenyl wiederum substituiert sein können mit 1-3 Substituenten aus der Gruppe F, Cl, Br, CF₃ bedeutet, und
R¹⁵ wie oben definiert ist,
und das andere Stereoisomere unverändert als Alkohol der Formel (IVa) 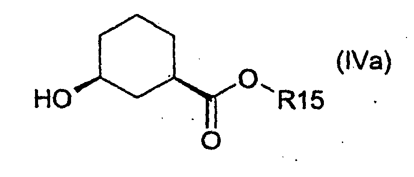 vorliegt, und daher durch Ausnutzung ihrer unterschiedlichen chemischen bzw. physikochemischen Eigenschaften voneinander getrennt werden können oder aber durch eine weiterführende chemische Folgereaktion, an der der Ester nicht teilnimmt, wobei
die als Alkohol anfallenden Enantiomere der Formel (IVa) wie unter d) beschrieben weiter verarbeitet werden, oder
c) Esterspaltung (ESp)
die als acylierte Verbindungen anfallenden Enantiomeren der Formel (Vb) zu chemisch enantiomeren Alkoholen (IVb) nach bekannten Verfahren verseift oder z. B. durch Umsetzung mit K₂CO₃ in Methanol zum optisch aktiven (1 S,5R)-6-Oxabicyclo[3.2.1]octan-7-on intramolekular umestert, welches in eine isomere Form des Produktes überführt werden kann 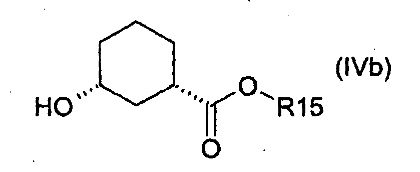 oder die Verbindung der Formel (Vb) z. B. durch lipasekatalysierte Spaltung beider Esterfunktionen zur optisch aktiven Verbindung der Formel (IVb, mit R¹⁵ = H) umwandelt, welche in eine isomere Form des Produktes überführt werden kann;
d) Alkylierung (Alk-R¹/ Alk-SG)
weiter mit Verbindungen der Formel (VI)
R¹-X (VI)
worin
R¹ 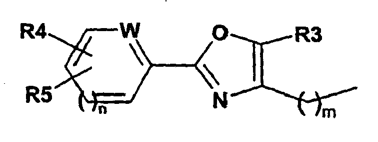 bedeutet und R³, R⁴, R⁵, W, n und m wie oben definiert sind, oder
R¹ für eine OH-Schutzgruppe (SG) steht wie oben definiert, mit Ausnahme von THP, EE, 1-Methyl-1-methoxyethyl oder 1-Methyl-1-benzyloxyethyl ;
und
X Cl, Br, I, OTs, OMs, OTf bedeutet;
in Gegenwart von Basen in einem geeigneten Lösungsmittel zu den Verbindungen der Formeln (VIIa) oder (VIIb); 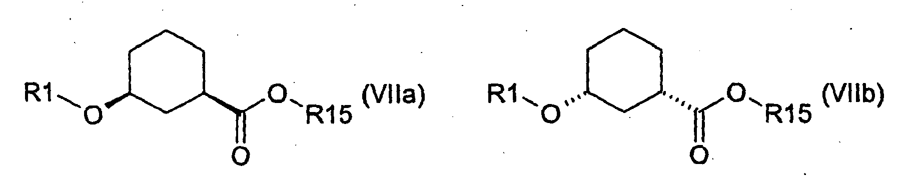 oder im Falle R¹ = SG zu den Verbindungen der Formeln (VIIIa) oder (VIIIb) 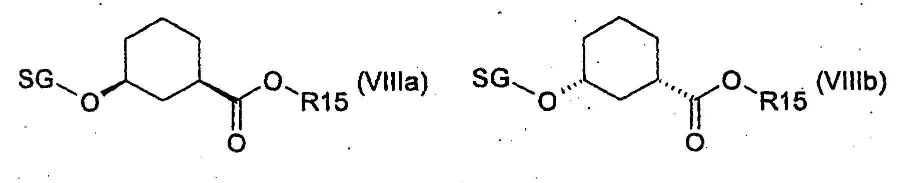 oder
R¹ für ein OH-Schutzgruppe (SG) steht wie Tetrahydropyranyl (THP), 1-Ethoxyethyl, 1-Methyl-1-methoxyethyl oder 1-Methyl-1-benzyloxyethyl steht;
und
zur Einführung der OH-Schutzgruppen die Verbindungen der Formel (IVa) und (IVb) säurekatalysiert mit den entsprechenden, bekannten Enolethern ebenfalls zu Verbindungen der Formeln (VIIIa) oder (VIIIb) umgesetzt werden;
und
e) direkte Umsetzung oder Esterspaltung & Kupplung (dU o. Sp + K)
e1) die erhaltenen Verbindungen der Formeln (VIIa) oder (VIIb) bzw. die Verbindungen der Formeln (VIIIa) oder (VIIIb) in einer direkten Umsetzung (dU), z. B. durch Reaktion eines Amins der Formel (IX)
R²-H (IX)
worin
R² 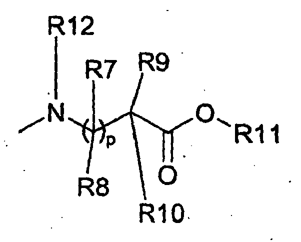 worin R7, R8, R9, R10, R11, R12 und p wie oben definiert sind,
bzw. dem korrespondierenden Lithium- bzw- Dimethylaluminium-Derivat, oder durch Reaktion der Verbindungen der Formeln (VIIa) oder (VIIb) bzw. der Verbindungen der Formeln (VIIIa) oder (VIIIb) und dem Amin bzw. Aminosäurederivat R²-H der Formel (IX) in Gegenwart aktivierender Reagenzien bzw. Katalysatoren in Verbindungen der Formeln (Ia) oder (Ib) oder deren isomere Formen überführt, 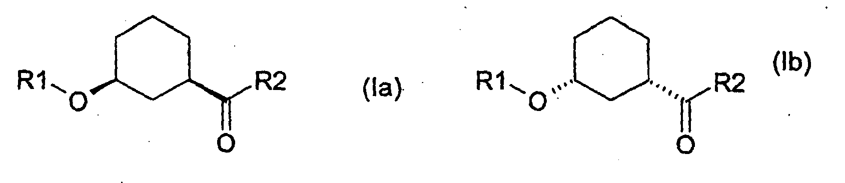 oder im Falle R¹ = SG in Verbindungen der Formeln (Xa) oder (Xb) überführt, 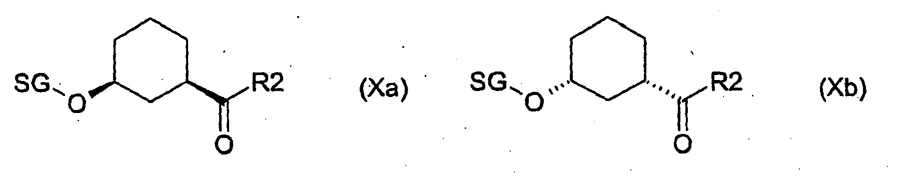 oder
e2) die erhaltenen Verbindungen der Formeln (VIIa) oder (VIIb) bzw. (VIIIa) oder (VIIIb) einer Esterspaltung , und die so erhaltenen Verbindungen der Formeln (XIa) oder (Xlb), bzw. (XIIIa) oder (XIIIb) 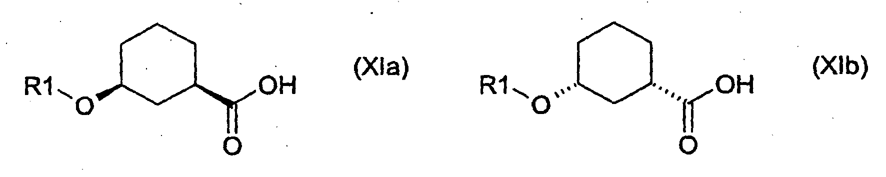 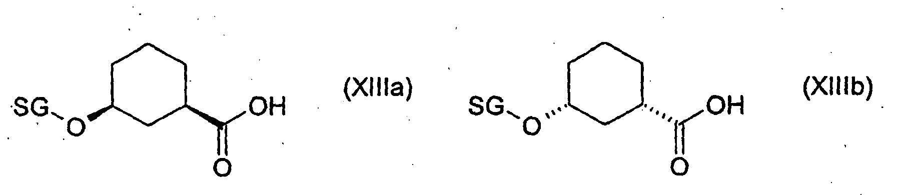 bzw. die entsprechenden Salze einer anschließender Kupplung unterwirft mit einer Verbindung der Formel (IX)
R²-H (IX)
worin
R² 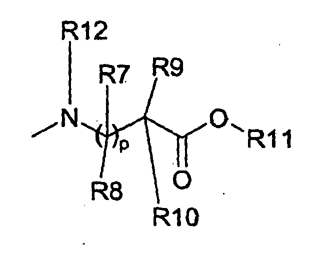 bedeutet und worin R7, R8, R9, R10, R11, R12 und p wie oben definiert sind,
in Gegenwart von wasserentziehenden oder aktivierenden Reagenzien zu einer Verbindung der Formeln (Ia) oder (Ib) oder deren isomere Formen; 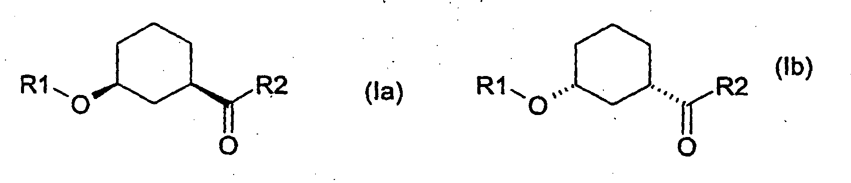 und gegebenenfalls
f) Abspaltung der Schutzgruppe SG (AbSG)
falls R¹ für eine OH-Schutzgruppe (SG) steht, wie oben unter R¹ definiert, werden die Verbindungen der Formeln (Xa) oder (Xb) 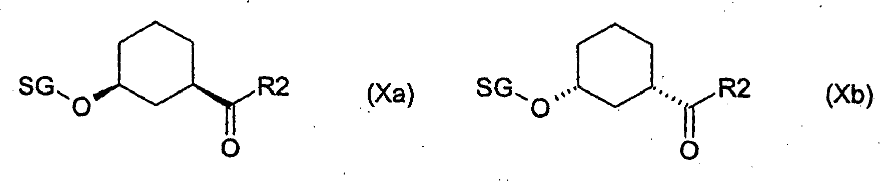 worin R² und SG wie oben definiert sind,
durch Abspaltung der Schutzgruppe nach bekannten Verfahren in Verbindungen der Formeln (Xlla) oder (XIIb) 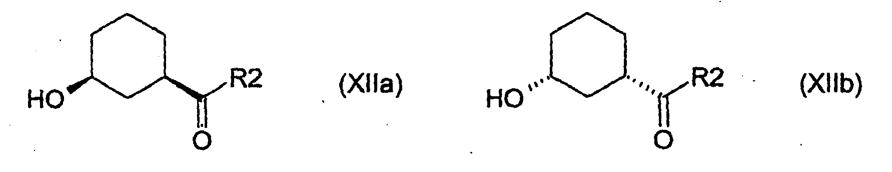 worin R² wie oben definiert ist, überführt, und dann entsprechend der angegebenen Verfahrensvarianten in die Verbindungen der Formeln (Ia) oder (Ib) oder deren isomere Formen umgewandelt;
wobei es auch möglich ist die Reihenfolge der einzelnen Reaktionsschritte wie vorstehend unter A) beschrieben:
**A)** LO → EB+T [→ ESp] → Alk-R¹ → dU oder Sp + K → Produkt/isomere Form
zu ändern in:
**B)** LO → EB+T [→ ESp] → dU oder Sp + K → Alk-R¹ → Produkt/ isomere Form
oder
**C)** LO → dU oder Sp + K → EB + T → [ESp] → Alk-R¹ → Produkt/ isomere Form
oder
**D)** LO → EB+T → [Esp] → Alk-SG → dU oder Sp + K → AbSG → Alk-R¹ → Produkt/ isomere Form,
oder
**E)** LO → Alk-SG → dU oder Sp +K → AbSG → EB + T → [ESp] → Alk-R¹ → Produkt/ isomere Form.

2. Verfahren gemäß Anspruch 1, **dadurch gekennzeichnet, dass** die Verfahren A), B) und D) angewendet werden.

3. Verfahren gemäß Anspruch 1 oder 2, **dadurch gekennzeichnet, dass** das Verfahren A) angewendet wird.

4. Verfahren zur Herstellung der Verbindungen der Formeln (Ia) und (Ib) gemäß den Ansprüchen 1 bis 3, **dadurch gekennzeichnet, daß**
R4 Br, CF₃, OCF₃, (C1-C6)-Alkyl, O-(C1-C6)-Alkyl;
R5 H, (C1-C6)-Alkyl, O-(C1-C6)-Alkyl oder
R4 und R5 zusammen mit dem Phenylring = Naphthyl;
R3 CF₃, (C1-C6)-Alkyl, (C3-C6)-Cycloalkyl, Phenyl;
W CH, falls n = 1;
m 1;
p 0
R9 H, (C1-C6)-Alkyl;
R10 (C1-C6)-Alkyl, wobei Alkyl gegebenenfalls substituiert sein kann durch Phenyl;
R10 und R12 zusammen mit den sie tragenden Atomen Pyrrolidin, falls p = 0;
R9 und R10 zusammen mit dem sie tragenden C-Atom (C3-C6)-Cycloalkyl;
R11 H,
R12 H, (C1-C6)-Alkyl, Benzyl.

## Claims

1. A process for preparing chiral nonracemic compounds of the formulae (Ia) and (Ib) 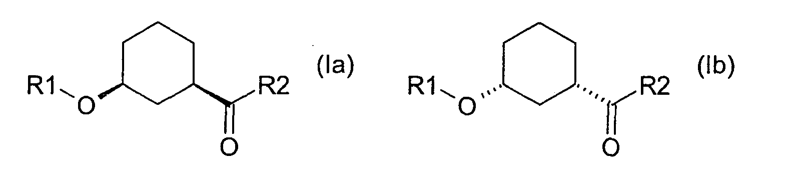 where:
R¹ is 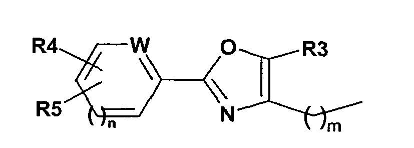 in which:
R³ is H, (C1-C6)-alkyl, (C3-C8)-cycloalkyl, (C1-C3)-alkyl-(C3-C8)-cycloalkyl, phenyl, (C1-C3)-alkyl-phenyl, (C5-C6)-heteroaryl, (C1-C3)-alkyl-(C5-C6)-heteroaryl or (C1-C3)-alkyl which is fully or partially substituted by F;
R⁴, R⁵ independently of one another are H, F, Cl, Br, CF₃, OCF₃, (C1-C6)-alkyl, O-(C1-C6)-alkyl, SCF₃, SF₅, OCF₂-CHF₂, (C6-C10)-aryl, (C6-C10)-aryloxy, OH, NO₂; or
R⁴ and R⁵ together with the carbon atoms that carry them form a fused partially saturated or unsaturated bicyclic (C6-C10)-aryl or (C5-C11)-heteroaryl ring;
W is CH, N, if n = 1;
W is O, S, NR⁶, if n = 0;
m is 1-6;
R⁶ is H, (C1-C6)-alkyl-phenyl, (C1-C6)-alkyl;
or
R¹ is an OH protective group (PG), such as, for example, benzyloxymethyl, benzyl, para-methoxybenzyl, tert-butyldimethylsilyl (TBDMS), tert-butyldiphenylsilyl (TBDPS), tetrahydropyranyl (THP), 1-ethoxyethyl (EE), 1-methyl-1-methoxyethyl or 1-methyl-1-benzyloxyethyl;
and
R² is 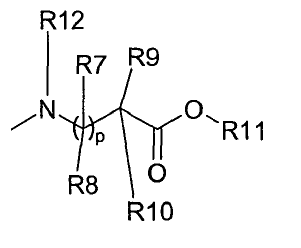 in which:
p is 0 - 2;
R⁷ is H, (C1-C6)-alkyl;
R⁸ is H, (C1-C6)-alkyl;
R⁹ is H, F, (C1-C6)-alkyl;
R¹⁰ is H, F, (C1-C6)-alkyl, O-(C1-C6)-alkyl, (C2-C6)-alkenyl, (C2-C6)-alkynyl, (C3-C8)-cycloalkyl, phenyl, where alkyl, alkenyl, alkynyl and cycloalkyl may optionally be substituted by one or more radicals from the group consisting of hydroxyl, phenyl, (C5-C11)-heteroaryl, O-(C1-C6)-alkyl and NR¹³R¹⁴, and phenyl may optionally be substituted by one or more radicals from the group consisting of hydroxyl, O-(C1-C6)-alkyl, F and CF₃, with the proviso that R¹⁰ is not NR¹³R¹⁴ or O-(C1-C6)-alkyl, if R⁹ = F;
R⁹ and R¹⁰ together with the carbon atom that carries them are (C3-C8)-cycloalkyl;
R¹⁰ and R¹² together are pyrrolidine and piperidine, if p = 0;
R¹¹ is H, (C1-C8)-alkyl, benzyl, (C1-C4)-alkyl-(C6-C10)-aryl, (C1-C4)-alkyl-O-(C1-C4)-alkyl, phenyl-(C1-C4)-alkyl, where alkyl, benzyl, phenyl, aryl may optionally be mono- or polysubstituted by O-(C1-C6)-alkyl, OCH₂CH₂-OMe, F, Cl, Br, I, Si(CH₃)₃, OSi(CH₃)₃, Si(iPr)₃, OSi(iPr)₃, OCH₂CH₂-SiMe₃, OCH₂-Si(iPr)₃, O-CH₂-C₆H₅, SO₂C₆H₄-p-Me, SMe, CN, NO₂, CH₂COC₆H₅;
R¹² is H, (C1-C6)-alkyl, (C2-C6)-alkenyl, (C2-C6)-alkynyl, benzyl, CO-(C1-C6)-alkyl, CO-phenyl, C(O)-O-(C1-C6)-alkyl, allyloxycarbonyl (ALOC), benzyloxycarbonyl (Cbz, Z), 9-fluorenylmethyloxycarbonyl (FMOC), (C1-C4)-alkyl-(C6-C10)-aryl, (C1-C4)-alkyl-(C5-C11)-heteroaryl, (C1-C4)-alkyl-O-(C1-C4)-alkyl, phenyl-(C1-C4)-alkyl, (C5-C6)-heteroaryl-(C1-C4)-alkyl; SO₂-(C1-C6)-alkyl, SO₂-(C1-C6)-alkyl-SO₂-(C1-C6)-alkyl, SO₂-phenyl, where phenyl may optionally be substituted by (C1-C6)-alkyl, O-(C1-C6)-alkyl, F, Cl;
R¹³ is (C1-C6)-alkyl;
R¹⁴ is (C1-C6)-alkyl-phenyl, (C1-C6)-alkyl;
wherein
**A)**
a) Lactone opening (LO)
racemic 6-oxabicyclo[3.2.1]octan-7-one of the formula (II) 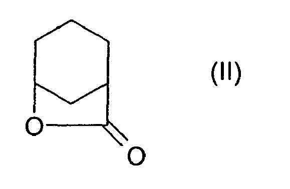 is reacted with a compound of the formula (III)
HO-R¹⁵ (III)
where
R¹⁵ is H, (C1-C8)-alkyl, (C3-C8)-cycloalkyl, (C2-C8)-alkenyl, (C2-C8)-alkynyl, benzyl, (C1-C4)-alkyl-(C6-C10)-aryl, (C1-C4)-alkyl-(C5-C11)-heteroaryl, (C1-C4)-alkyl-O-(C1-C4)-alkyl, phenyl-(C1-C4)-alkyl, (C5-C6)-heteroaryl-(C1-C4)-alkyl, where alkyl, benzyl, phenyl, aryl, heteroaryl may optionally be mono- or polysubstituted by phenyl, O-(C1-C6)-alkyl, OCH₂CH₂-OMe, OTs, F, Cl, Br, I, Si(CH₃)₃, OSi(CH₃)₃, Si(iPr)₃, OSi(iPr)₃, OCH₂CH₂-SiMe₃, OCH₂-Si(iPr)₃, OTHP, O-CH₂-C₆H₅, SO₂C₆H₄-p-Me, SMe, CN, NO₂, COOH, CONH₂, CH₂COC₆H₅, CO-benzyloxy, CO-O(C1-C6)-alkyl, NHTs, NHAc, NHBoc, NHAloc, NHbenzyl;
in the presence of suitable bases or acids in a suitable solvent or with acid halides in alcohols, where in the case of aqueous reactions the work-up conditions determine whether the salt or else the free acid is obtained, to give, for example, a racemic cis-configured compound of the formula (IV), 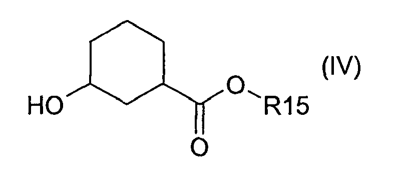 in which R¹⁵ is as defined above, or to give a compound of the formula (IV) which, depending on the work-up, may be present in ionic form, for example as Cs⁺, Li⁺, K⁺, NH₄⁺, Ca²⁺, Ba²⁺, Mg²⁺ salt and in which R¹⁵ is also Cs, Li, K, NH₄, Ca, Ba, Mg, and, if appropriate, the resulting product is further converted into another product of the formula (IV);
b) Enzymatic ester formation (EF) + separation (S)
the resulting compounds of the formula (IV) are subjected to a stereoselective enzymatic ester formation (EF), where an acyl donor and the enzyme from the group of the hydrolases are added to the hydroxyl compounds in an organic solvent and the resulting mixture is stirred at from -20 to 80°C and, after the reaction has ended, one stereoisomer is present as ester of the formula (Vb) 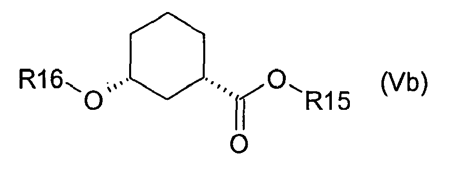 in which
R¹⁶ is C(=O)-(C₁-C₁₆)-alkyl, C(=O)-(C₂-C₁₆)-alkenyl, C(=O)-(C₃-C₁₆)-alkynyl, C(=O)-(C₃-C₁₆)-cycloalkyl, where one or more carbon atoms may be replaced by oxygen atoms and may be substituted by 1-3 substituents from the group consisting of F, Cl, Br, CF₃, CN, NO₂, hydroxyl, methoxy, ethoxy, phenyl, CO-O(C₁-C₄)-alkyl and CO-O(C₂-C₄)-alkenyl, where phenyl, CO-O(C₁-C₄)-alkyl and CO-O(C₂-C₄)-alkenyl for their part may be substituted by 1-3 substituents from the group consisting of F, Cl, Br, CF₃, and
R¹⁵ is as defined above,
and the other stereoisomer is present unchanged as the alcohol of the formula (IVa) 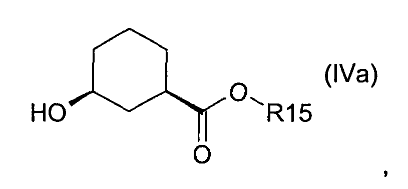 which compounds can therefore, utilizing their different chemical or physicochemical properties, be separated from one another or else be processed further by a subsequent chemical follow-up reaction in which the ester does not participate, where
the enantiomers of the formula (IVa) obtained as alcohol are processed further as described under d), or
c) Ester cleavage (EC)
the enantiomers of the formula (Vb) obtained as acylated compounds are hydrolyzed by known processes to give chemically enantiomeric alcohols (IVb) or, for example by reaction with K₂CO₃ in methanol, transesterified intramolecularly to give the optically active (1S,5R)-6-oxabicyclo[3.2.1]octan-7-one which can be converted into an isomeric form of the product 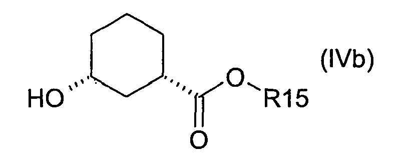 or the compound of the formula (Vb) is converted, for example, by lipase-catalyzed cleavage of both ester functions into the optically active compound of the formula (IVb, where R¹⁵ = H) which can be converted into an isomeric form of the product;
d) Alkylation (Alk-R¹/Alk-PG)
further conversion with compounds of the formula (VI)
R¹-X (VI)
in which
R¹ is 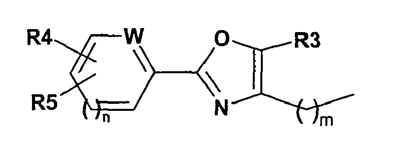 and R³, R⁴, R⁵, W, n and m are as defined above, or
R¹ is an OH protective group (PG) as defined above, except for THP, EE, 1-methyl-1-methoxyethyl or 1-methyl-1-benzyloxyethyl;
and
X is Cl, Br, I, OTs, OMs, OTf;
in the presence of bases in a suitable solvent to give compounds of the formula (VIIa) or (VIIb); 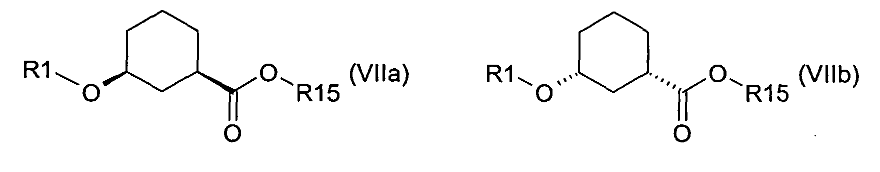 or, in the case of R¹ = PG, to give compounds of the formula (VIIIa) or (VIIIb) 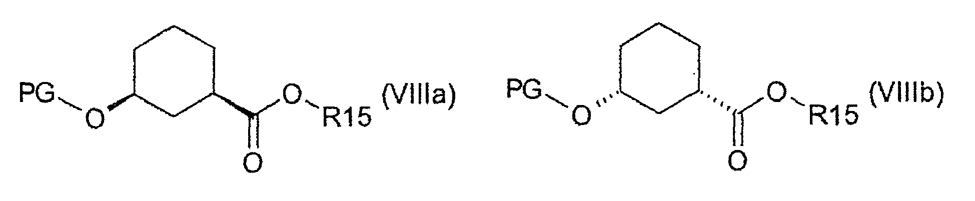 or
R¹ is an OH protective group (PG) such as tetrahydropyranyl (THP), 1-ethoxyethyl, 1-methyl-1-methoxyethyl or 1-methyl-1-benzyloxyethyl;
and
to introduce the OH protective groups, the compounds of the formula (IVa) or (IVb) are reacted under acid catalysis with the appropriate known enol ethers, also to give compounds of the formula (VIIIa) or (VIIIb);
and
e) Direct reaction or ester cleavage & coupling (DR or EC+C)
e1) the resulting compounds of the formula (VIIa) or (VIIb) or compounds of the formula (VIIIa) or (VIIIb) are converted in a direct reaction (DR), for example by reacting an amine of the formula (IX)
R²-H (IX)
in which
R² is 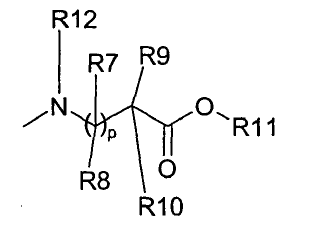 where R7, R8, R9, R10, R11, R12 and p are as defined above,
or the corresponding lithium or dimethylaluminum derivative, or by reacting the compounds of the formula (VIIa) or (VIIb) or the compounds of the formula (VIIIa) or (VIIIb) and the amine or amino acid derivative R²-H of the formula (IX) in the presence of activating reagents or catalysts, to give compounds of the formula (Ia) or (Ib) or isomeric forms thereof, 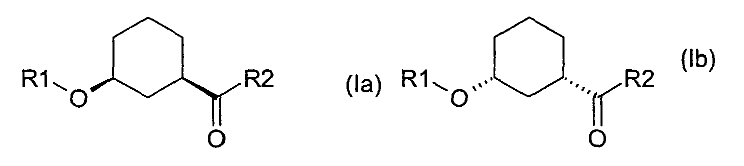 or, in the case of R¹ = PG, to give compounds of the formula (Xa) or (Xb), 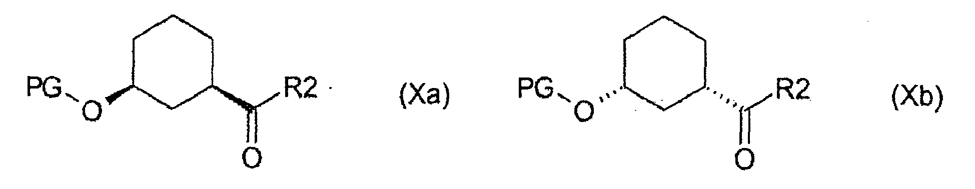 or
e2) the resulting compounds of the formula (VIIa) or (VIIb) or (VIIIa) or (VIIIb) are subjected to an ester cleavage, and the resulting compounds of the formula (XIa) or (XIb) or (XIIIa) or (XIIIb) 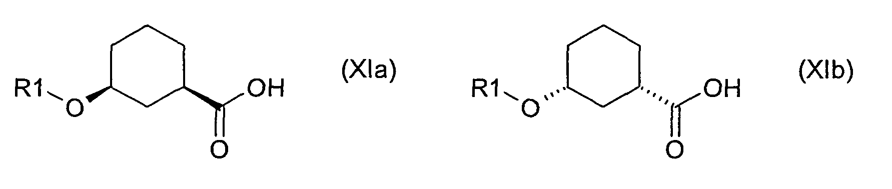 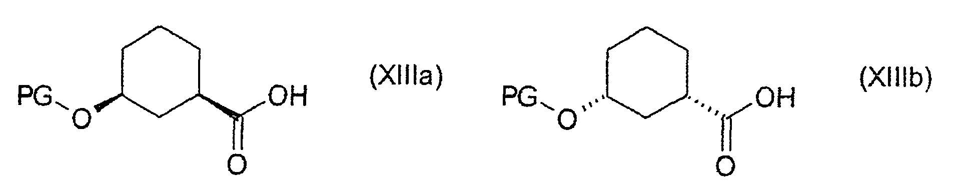 or the corresponding salts are subjected to a subsequent coupling with a compound of the formula (IX)
R²-H (IX)
in which
R² is 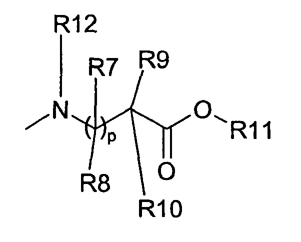 where R7, R8, R9, R10, R11, R12 and p are as defined above,
in the presence of dehydrating or activating reagents, to give a compound of the formula (Ia) or (Ib) or an isomeric form thereof; 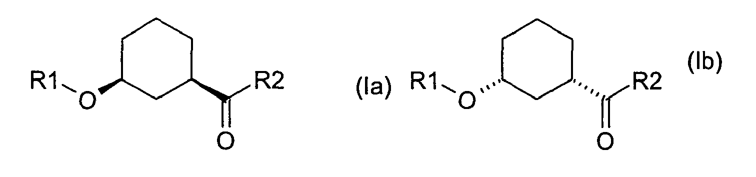 and, if appropriate,
f) Removal of the protective group PG (RPG)
if R¹ is an OH protective group (PG) as defined above under R¹, the compounds of the formula (Xa) or (Xb) 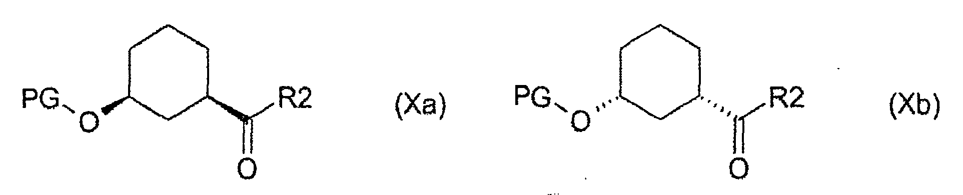 in which R² and PG are as defined above
are converted by removal of the protective group by known processes into compounds of the formula (Xlla) or (XIIb) 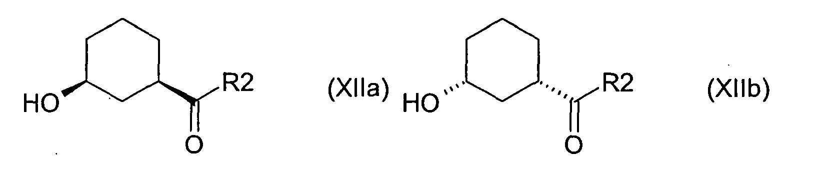 in which R² is as defined above, followed by conversion according to the stated process variants into the compounds of the formula (Ia) or (Ib) or isomeric forms thereof;
it also being possible to change the sequence of the individual reaction
steps as described above under A):
**A)** LO → EF+S [→ EC] → Alk-R¹ → DR or EC + C → product/isomeric form
to:
**B)** LO → EF+S [→ EC] → DR or EC + C → Alk-R¹ → product/isomeric form
or
**C)** LO → DR or EC + C → EF + S → [EC] → Alk-R¹ → product/isomeric form
or
**D)** LO → EF+S → [EC] → Alk-PG → DR or EC + C → RPG → Alk-R¹ → product/isomeric form,
or
**E)** LO → Alk-PG → DR or EC + C → RPG → EF + S → [EC] → Alk-R¹ → product/isomeric form.

2. The process as claimed in claim 1, wherein the processes A), B) and D) are employed.

3. The process as claimed in claim 1 or 2, wherein process A) is employed.

4. The process for preparing the compounds of the formulae (Ia) and (Ib) as claimed in any of claims 1 to 3, wherein
R4 is Br, CF₃, OCF₃, (C1-C6)-alkyl, O-(C1-C6)-alkyl;
R5 is H, (C1-C6)-alkyl, O-(C1-C6)-alkyl or
R4 and R5 together with the phenyl ring = naphthyl;
R3 is CF₃, (C1-C6)-alkyl, (C3-C6)-cycloalkyl, phenyl;
W is CH, if n = 1;
m is 1;
p is 0;
R9 is H, (C1-C6)-alkyl;
R10 is (C1-C6)-alkyl, where alkyl may optionally be substituted by phenyl;
R10 and R12 together with the atoms that carry them are pyrrolidine, if p = 0;
R9 and R10 together with the carbon atom that carries them are (C3-C6)-cycloalkyl;
R11 is H,
R12 is H, (C1-C6)-alkyl, benzyl.

## Revendications

1. Procédé de préparation de composés chiraux, non racémiques des formules (Ia) et (Ib) 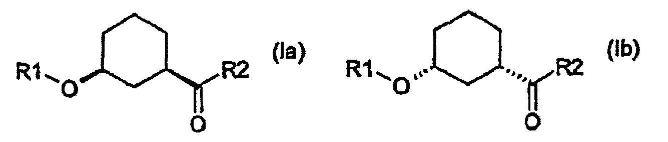 avec
R¹ signifiant 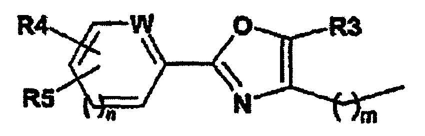 dans laquelle :
R³ signifie H, (C₁-C₆)-alkyle, (C₃-C₈)-cycloalkyle, (C₁-C₃) -alkyl- (C₃-C₈) -cycloalkyle, phényle, (C₁-C₃)-alkyl-phényle, (C₅-C₆)-hétéroaryle, (C₁-C₃)-alkyl-(C₅-C₆)-hétéroaryle ou (C₁-C₃)-alkyle, qui est totalement ou partiellement substitué par F ;
R⁴, R⁵ signifient, indépendamment l'un de l'autre, H, F, Cl, Br, CF₃, OCF₃, (C₁-C₆)-alkyle, O-(C₁-C₆)-alkyle, SCF₃, SF₅, OCF₂-CHF₂, (C₆-C₁₀)-aryle, (C₆-C₁₀)-aryloxy, OH, NO₂ ; ou
R⁴ et R⁵ pris ensemble avec les atomes de carbone qui les portent, forment un cycle (C₆-C₁₀)-aryle ou (C₅-C₁₁)-hétéroaryle bicyclique condensé, partiellement saturé ou non sature ;
W signifie CH, N, si n = 1 ;
W signifie O, S, NR⁶, si n = 0 ;
m vaut 1-6 ;
R⁶ signifie H, (C₁-C₆)-alkyl-phényle, (C₁-C₆)alkyle ;
ou
R¹ signifie un groupe de protection de la fonction OH (GP), tel que par exemple benzyloxyméthyle, benzyle, para-méthoxybenzyle, tert-butyldiméthylsilyle (TBDMS), tert-butyldiphénylsilyle (TBDPS), tétrahydropyrannyle (THP), 1-éthoxyéthyle (EE), 1-méthyl-1-méthoxyéthyle ou 1-méthyl-1-benzyloxyéthyle ;
et
R² signifiant 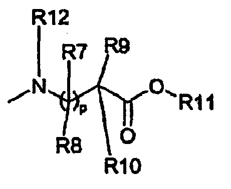 dans laquelle :
p vaut 0-2 ;
R⁷ signifie H, (C₁-C₆)-alkyle ;
R⁸ signifie H, (C₁-C₆)-alkyle ;
R⁹ signifie H, F, (C₁-C₆)-alkyle ;
R¹⁰ signifie H, F, (C₁-C₆)-alkyle, O-(C₁-C₆)-alkyle, (C₂-C₆)-alcényle, (C₂-C₆)-alcynyle, (C₃-C₈)-cycloalkyle, phényle, où alkyle, alcényle, alcynyle et cycloalkyle peuvent le cas échéant être substitués par un ou plusieurs radicaux de la série hydroxy, phényle, (C₅-C₁₁)-hétéroaryle, O-(C₁-C₆)-alkyle et NR¹³R¹⁴, et phényle peut le cas échéant être substitué par un ou plusieurs radicaux de la série hydroxy, O-(C₁-C₆)-alkyle, F et CF₃, à condition que R¹⁰ soit différent de NR¹³R¹⁴ ou de O-(C₁-C₆)-alkyle, lorsque R⁹ = F ;
R⁹ et R¹⁰ signifient, ensemble avec l'atome de C qui les porte, (C₃-C₈)-cycloalkyle ;
R¹⁰ et R¹² signifient, ensemble, pyrrolidine ou pipéridine, si p = 0 ;
R¹¹ signifie H, (C₁-C₈)-alkyle, benzyle, (C₁-C₄)-alkyl-(C₆-C₁₀) -aryle, (C₁-C₄) -alkyl-O- (C₁-C₄) -alkyle, phényl-(C₁-C₄)-alkyle, où alkyle, benzyle, phényle, aryle peuvent le cas échéant être monosubstitués ou polysubstitués par O-(C₁-C₆)-alkyle, OCH₂CH₂-OMe, F, Cl, Br, I, Si(CH₃)₃, OSi(CH₃)₃, Si(iPr)₃, OSi(iPr)₃, OCH₂CH₂-SiMe₃, OCH₂-Si(iPr)₃, O-CH₂-C₆H₅, SO₂C₆H₄-p-Me, SMe, CN, NO₂, CH₂COC₆H₅ ;
R¹² signifie H, (C₁-C₆)-alkyle, (C₂-C₆)-alcényle, (C₂-C₆)-alcynyle, benzyle, CO-(C₁-C₆) -alkyle, CO-phényle, C(O)-O-(C₁-C₆)-alkyle, allyloxycarbonyle (ALOC), benzyloxycarbonyle (Cbz, Z), 9-fluorénylméthyloxycarbonyle (FMOC), (C₁-C₄)-alkyl-(C₆-C₁₀) -aryle, (C₁-C₄) -alkyl- (C₅-C₁₁) -hétéroaryle, (C₁-C₄)-alkyl-O- (C₁-C₄) -alkyle, phényl-(C₁-C₄)-alkyle, (C₅-C₆)-hétéroaryl-(C₁-C₄)-alkyle ; SO₂-(C₁-C₆)-alkyle, SO₂- (C₁-C₆)alkyl-SO₂- (C₁-C₆) -alkyle, SO₂-phényle, où phényle peut le cas échéant être substitué par (C₁-C₆)-alkyle, O-(C₁-C₆)-alkyle, F, Cl ;
R¹³ signifie (C₁-C₆)-alkyle ;
R¹⁴ signifie (C₁-C₆) -alkyl-phényle, (C₁-C₆)-alkyle ;
**caractérisé en ce que**
A)
a) Ouverture de lactone (OL)
on transforme de la 6-oxabicyclo[3.2.1]octan-7-one racémique de formule (II) 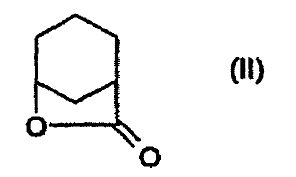 avec un composé de formule (III)
HO-R¹⁵ (III)
avec
R¹⁵ signifiant H, (C₁-C₈)-alkyle, (C₃-C₈)-cycloalkyl (C₂-C₈) -alcényle, (C₂-C₈)-alcynyle, benzyle, (C₁-C₄)-alkyl-(C₆-C₁₀)-aryle, (C₁-C₄)-alkyl-(C₅-C₁₁)-hétéroaryle, (C₁-C₄)-alkyl-O- (C₁-C₄) -alkyle, phényl-(C₁-C₄) -alkyle, (C₅-C₆) -hétéroaryl- (C₁-C₄)-alkyle, où alkyle, benzyle, phényle, aryle, hétéroaryle peuvent le cas échéant être monosubstitués ou polysubstitués par phényle, O-(C₁-C₆)-alkyle, OCH₂CH₂-OMe, OTs, F, Cl, Br, I, Si(CH₃)₃, OSi(CH₃)₃, Si(iPr)₃, OSi(iPr)₃, OCH₂CH₂-SiMe₃, OCH₂-Si(iPr)₃, OTHP, O-CH₂-C₆H₅, SO₂C₆H₄-p-Me, SMe, CN, NO₂, COOH, CONH₂, CH₂COC₆H₅, CO-benzoxy, CO-O(C₁-C₆)-alkyle, NHTs, NHAc, NHBoc, NHAloc, NHbenzyle ;
en présence de bases ou d'acides appropriés dans un solvant approprié ou avec des halogénures d'acide dans des alcools, où, dans le cas de réactions aqueuses, les conditions du traitement sont déterminantes pour l'obtention du sel ou de l'acide libre, par exemple en un composé racémique à configuration cis de formule (IV), 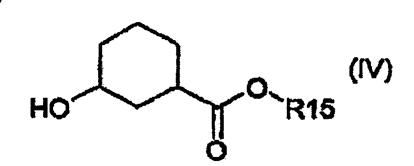 où R¹⁵ est tel que défini comme ci-dessus, ou en un composé de formule (IV), qui, en fonction du traitement, peut se trouver sous forme ionique, par exemple sous forme de sel de Cs⁺, Li⁺, K⁺, NH₄⁺, Ca²⁺, Ba²⁺, Mg²⁺ et où R¹⁵ signifie également Cs, Li, K, NH₄, Ca, Ba, Mg, et on transforme le cas échéant le produit ainsi obtenu en un autre produit de formule (IV) ;
b) Formation enzymatique d'un ester (FE) et séparation (S)
on soumet les composés obtenus de formule (IV) à une formation d'ester (FE) enzymatique stéréosélective, les composés hydroxy étant mélangés dans un solvant organique avec un donneur d'acyle et l'enzyme du groupe des hydrolases et le mélange obtenu étant agité à -20 jusqu'à 80°C et, après la réaction, un stéréo-isomère se trouve sous forme d'ester de formule (Vb) 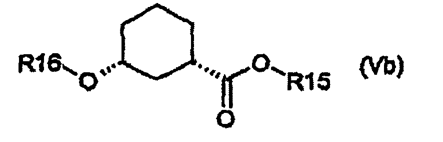 où
R¹⁶ signifie C(=O)-(C₁-C₁₆)-alkyle, C(=O)-(C₂-C₁₆)-alcényle, C(=O)-(C₃-C₁₆)-alcynyle, C(=O)-(C₃-C₁₆)-cycloalkyle, un ou plusieurs atomes de carbone pouvant être remplacés par des atomes d'oxygène et pouvant être substitués par 1-3 substituants du groupe F, Cl, Br, CF₃, CN, NO₂, hydroxy, méthoxy, éthoxy, phényle, CO-O(C₁-C₄)-alkyle et CO-O(C₂-C₄)-alcényle, où phényle, CO-O(C₁-C₄)-alkyle et CO-O(C₂-C₄)-alcényle peuvent être substitués à leur tour par 1-3 substituants du groupe F, Cl, Br, CF₃, et
R¹⁵ est défini comme ci-dessus,
et l'autre stéréo-isomère se trouve sous forme non modifiée comme alcool de formule (IVa) 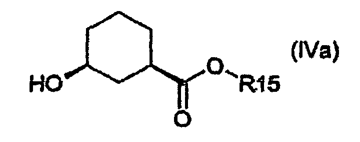 et ils peuvent dès lors être séparés l'un de l'autre en exploitant leurs propriétés chimiques ou physicochimiques différentes ou également par une réaction chimique consécutive plus poussée, à laquelle l'ester ne participe pas, où
les énantiomères produits sous forme d'alcool de formule (IVa) sont transformés davantage comme décrit ci-dessous au point d), ou
c) Dissociation d'ester (DE)
on saponifie les énantiomères produits sous forme de composés acylés de formule (Vb) en alcools chimiquement énantiomères (IVb) selon des procédés connus ou on les transestérifie de manière intramoléculaire par exemple par transformation avec du K₂CO₃ dans du méthanol en (1S,5R)-6-oxabicyclo[3.2.1]octan-7-one optiquement active, qui peut être transformée en une forme isomère du produit 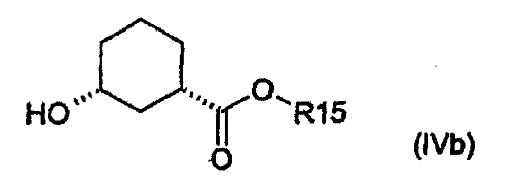 ou on transforme le composé de formule (Vb) par exemple par clivage catalysé par une lipase des deux fonctions ester en composé optiquement actif de formule (IVb, avec R¹⁵ = H), qui peut être transformé en une forme isomère du produit ;
d) Alkylation (alk-R¹/alk-GP)
on transforme ensuite avec des composés de formule (VI)
R¹-X (VI)
où
R¹ signifie 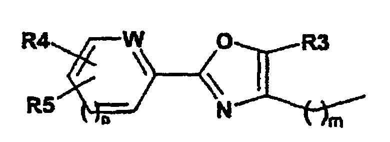 et R³, R⁴, R⁵, W, n et m sont définis comme ci-dessus ou
R¹ représente un groupe de protection de la fonction OH (GP) tel que défini ci-dessus, à l'exception de THP, EE, 1-méthyl-1-méthoxyéthyle ou 1-méthyl-1-benzyloxyéthyle ;
et
X signifie Cl, Br, I, OTs, OMs, OTf ;
en présence de bases dans un solvant approprié en composés des formules (VIIa) ou (VIIb) ; 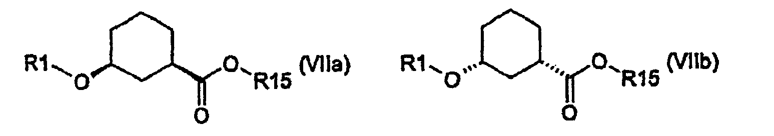 ou dans le cas où R¹ = GP, en composés des formules (VIIIa) ou (VIIIb) 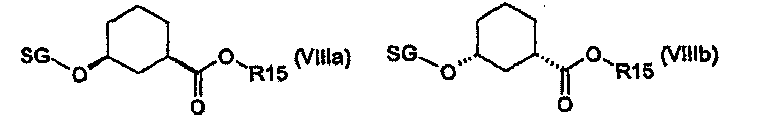 ou
R¹ signifie un groupe de protection de la fonction OH (GP), tel que tétrahydropyrannyle (THP), 1-éthoxyéthyle, 1-méthyl-1-méthoxyéthyle ou 1-méthyl-1-benzyloxyéthyle ;
et
pour l'introduction des groupes de protection de la fonction OH, on transforme les composés de formule (IVa) et (IVb) sous catalyse acide avec les énoléthers connus correspondants, également en composés des formules (VIIIa) ou (VIIIb) ;
et
e) Transformation directe ou dissociation d'ester & couplage (TD ou D + C)
e1) on transforme les composés obtenus des formules (VIIa) ou (VIIb) ou les composés des formules (VIIIa) ou (VIIIb) dans une transformation directe (TD), par exemple par réaction d'une amine de formule (IX)
R²-H (IX)
où
R² signifie 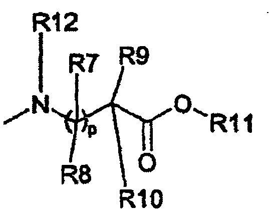 dans laquelle R⁷, R⁸, R⁹, R¹⁰, R¹¹, R¹² et p sont définis comme ci-dessus,
ou le dérivé de lithium ou de diméthylaluminium correspondant, ou par réaction des composés des formules (VIIa) ou (VIIb) ou les composés des formules (VIIIa) ou (VIIIb) et l'amine ou le dérivé d'acide aminé R²-H de formule (IX) en présence de réactifs d'activation ou de catalyseurs en composés des formules (Ia) ou (Ib) ou leurs formes isomères, 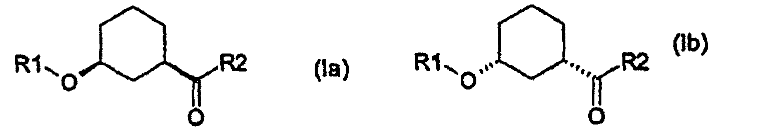 ou, dans le cas où R¹ = GP, en composés des formules (Xa) ou (Xb) 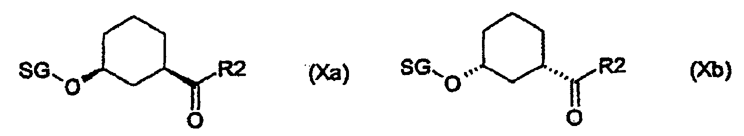 ou
e2) on soumet les composés obtenus des formules (VIIa) ou (VIIb) ou, selon le cas, (VIIIa) ou (VIIIb) à une dissociation d'ester, et les composés ainsi obtenus des formules (XIa) ou (XIb), ou selon le cas, (XIIIa) ou (XIIIb) 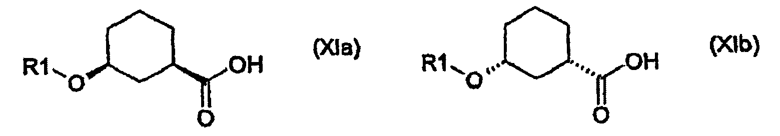 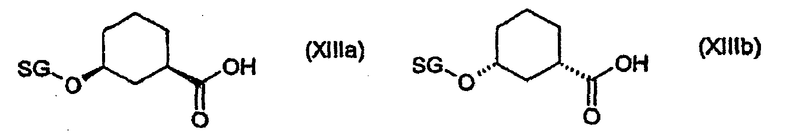 ou les sels correspondants à un couplage consécutif avec un composé de formule (IX)
R²-H (IX)
où
R² signifie 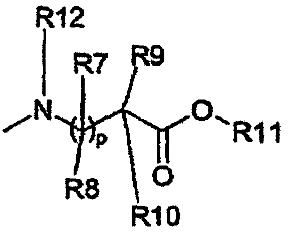 et dans laquelle R⁷, R⁸, R⁹, R¹⁰, R¹¹, R¹² et p sont définis comme ci-dessus,
en présence de réactifs retirant de l'eau ou d'activation en un composé des formules (Ia) ou (Ib) ou leurs formes isomères ; 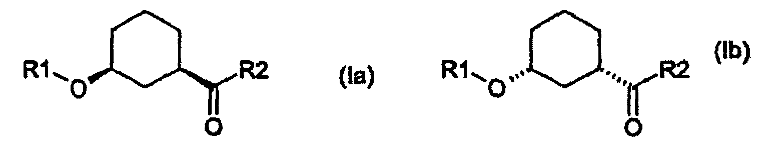 et le cas échéant
f) dissociation du groupe de protection GP (diGP) lorsque R¹ représente un groupe de protection de la fonction OH (GP) tel que défini ci-dessus pour R¹, on transforme les composés des formules (Xa) ou (Xb) 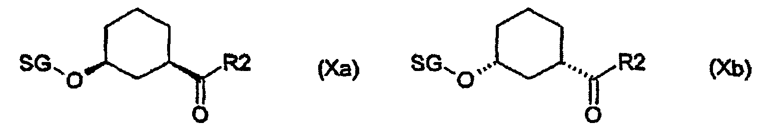 dans laquelle R² et GP sont définis comme ci-dessus, par dissociation du groupe de protection selon des procédés connus en composés des formules (XIIa) ou (XIIb) 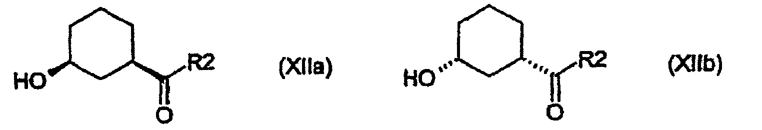 où R² est tel que défini ci-dessus, puis on les transforme selon les variantes de procédé indiquées en composés des formules (Ia) ou (Ib) ou leurs formes isomères ;
où il est également possible de modifier l'ordre des différentes étapes de réaction tel que décrit ci-dessus au point A) :
A) OL → FE + S [→ DE] → alk-R¹ → TD ou D + C → produit/forme isomère
en
B) OL → FE + S [→ DE] → TD ou D + C → alk-R¹ → produit/forme isomère
ou
C) OL → TD ou D + C → FE + S → [DE] → alk-R¹ → produit/forme isomère
ou
D) OL → FE + S → [DE] → alk-GP → TD ou D + Cl → diGP → alk-R¹ → produit/forme isomère,
ou
E) OL → alk-GP → TD ou D + C → diGP → FE + S → [DE] → alk-R¹ → produit/forme isomère.

2. Procédé selon la revendication 1, **caractérisé en ce qu'**on utilise les procédés A), B) et D).

3. Procédé selon la revendication 1 ou 2, **caractérisé en ce qu'**on utilise le procédé A).

4. Procédé pour la préparation des composés des formules (Ia) et (Ib) selon les revendications 1 et 3, **caractérisé en ce que**
R⁴ signifie Br, CF₃, OCF₃, (C₁-C₆)-alkyle, O-(C₁-C₆)-alkyle ;
R⁵ signifie H, (C₁-C₆)-alkyle, O-(C₁-C₆)-alkyle ou
R⁴ et R⁵ ensemble avec le cycle phényle = naphtyle ;
R³ signifie CF₃, (C₁-C₆)-alkyle, (C₃-C₆) -cycloalkyle, phényle ;
W signifie CH, si n = 1 ;
m vaut 1 ;
p vaut 0 ;
R⁹ signifie H, (C₁-C₆)-alkyle ;
R¹⁰ signifie (C₁-C₆)-alkyle, où alkyle peut le cas échéant être substitué par phényle ;
R¹⁰ et R¹² signifient, ensemble avec les atomes qui les portent, pyrrolidine, si p = 0 ;
R⁹ et R¹⁰ signifient, ensemble avec l'atome de C qui les porte, (C₃-C₆)-cycloalkyle ;
R¹¹ signifie H,
R¹² signifie H, (C₁-C₆)-alkyle, benzyle.
